# EUROPEAN PATENT SPECIFICATION

(11) **EP 2 941 176 B1**
(45) Date of publication and mention of the grant of the patent: **24.04.2019**
(21) Application number: 13870109.9
(22) Date of filing: 20.12.2013
(51) Int. Cl.: A61B 1/273, A61B 5/01, A61B 5/00, A61B 18/00, A61B 18/04, G01J 5/08

(54) **TEMPERATURE MEASUREMENT SYSTEM**
TEMPERATURMESSSYSTEM
SYSTÈME DE MESURE DE LA TEMPÉRATURE

(30) Priority: 07.01.2013 US 201361749617 P
(43) Date of publication of application: 11.11.2015
(73) Proprietor: Securus Medical Group, Inc., Cleveland, Ohio 44106 (US)
(72) Inventor: GARIBOTTO, John T., Marblehead, Massachusetts 01945 (US); AUGER, Steven T., Cohasset, Massachusetts 02025 (US); FLAHERTY, R. Maxwell, Auburndale, Florida 33823 (US); FLAHERTY, J. Christopher, Auburndale, Florida 33823 (US)
(74) Representative: Pfenning, Meinig & Partner mbB
(86) International application number: PCT/US2013/076961
(87) International publication number: WO 2014/107341

(56) References cited:
- EP-A1- 1 717 620
- WO-A2-2009/120694
- WO-A2-2012/071388
- US-A1- 2002 076 178
- US-A1- 2011 270 080
- US-A1- 2012 071 824
- US-A1- 2012 232 534

## Description

### Related Applications

This patent application is related to International Patent Application Serial Number PCT/US2011/061802, entitled "Ablation and Temperature Measurement Devices", filed November 22, 2011. This application claims the benefit of United States Provisional Application Serial No. 61/749,617, filed January 7, 2013.

### Field

Embodiments relate generally to the field of tissue temperature monitoring, and more particularly, to ablation and temperature measurement devices and systems that monitor tissue temperature during energy delivery.

### BACKGROUND

Numerous medical procedures include the delivery of energy to change the temperature of target tissue, such as to ablate or otherwise treat the tissue. With today's energy delivery systems, such as disclosed in document WO2012/071388, it is difficult for an operator of the system, such as a clinician, to treat all of the target tissue while avoiding adversely affecting non-target tissue. In treatment of a cardiac arrhythmia, ablation of heart tissue can often ineffectively ablate target tissue such as heart wall tissue, while inadvertently ablating esophageal tissue. In tumor ablation procedures, cancerous tissue ablation may also be incomplete or healthy tissue may be damaged.

There is a need for energy delivery and energy monitoring systems which allow a clinician to properly deliver energy to target tissue, while avoiding any destructive energy delivery to non-target tissue.

### SUMMARY

According to a first aspect, a system for producing surface temperature estimations of a tissue surface comprises a first optical assembly constructed and arranged to receive infrared light emitted from multiple tissue surface areas; a fiber comprising a proximal end and a distal end, where the distal end is optically coupled to receive infrared light from the first optical assembly; and a sensor optically coupled to the fiber proximal end, where the sensor is constructed and arranged to produce a signal that correlates to an average temperature of each of the multiple tissue surface areas.

The system can be constructed and arranged to produce surface temperature estimations of a surface of an esophagus.

The system can further comprise a probe comprising the first optical assembly and the fiber. The probe diameter can be less than or equal to 15F, or less than or equal to 12F, or less than or equal to 9F, or less than or equal to 6Fr.

The first optical assembly can comprise a surrounding tube. The surrounding tube can comprise a relatively infrared transmissive tube. The surrounding tube can comprise a material selected from the group consisting of: density polyethylene (HDPE) or low density polyethylene (LDPE); germanium; and combinations of these.

The first optical assembly can comprise an optical element selected from the group consisting of: optical fiber; lens; mirror; filter; prism; amplifier; refractor; splitter; polarizer; aperture; and combinations of these.

The first optical assembly can comprise an optical element constructed and arranged to perform an action on the received infrared light selected from the group consisting of: focus; split; filter; transmit without filtering; amplify; refract; reflect; polarize; and combinations of these.

The first optical assembly can comprise a rigid length less than or equal to 3cm, or less than or equal to 2cm, or less than or equal to 1cm, or less than or equal to 0.5cm.

The first optical assembly can comprise an optical element comprising a planar surface, an angled surface and a convex surface. The angled surface can comprise an angle of approximately 45°. The convex surface can comprise a convex surface with approximately a 4mm radius.

The first optical assembly can comprise an optical element with a first surface constructed and arranged to receive infrared light from tissue and a second surface constructed and arranged to direct the received infrared light to the fiber distal end. The second surface can comprise a convex surface. The first optical assembly can comprise an optical separation distance between the second surface and the fiber distal end. In some embodiments, the fiber can comprise an approximately 400µm core, the optical separation distance can comprise a distance of approximately 4.5mm, and the second surface can comprise a convex radius of approximately 3mm. In this embodiment, the first optical assembly can comprise a focal length of approximately 3.5mm, and the system can be constructed and arranged to receive infrared light from multiple tissue surface areas comprising an area of approximately 0.4mm. In some embodiments, the fiber can comprise an approximately 400µm core, the optical separation distance can comprise a distance of approximately 4.2mm, and the second surface can comprise a convex radius of approximately 4mm. In this embodiment, the first optical assembly can comprise a focal length of approximately 7.5mm, the system can be constructed and arranged to receive infrared light from multiple tissue surface areas comprising an area of approximately 1.0mm, and the system can comprise spatial resolution criteria correlating to a depth of field of approximately 8mm.

The first optical assembly can comprise a focal length of less than or equal to 10mm, or less than or equal to 5mm, for example, a focal length of approximately 3.2mm, or approximately 3.5mm. The first optical assembly can comprise a focal length between 4mm and 10mm.

The system can comprise spatial resolution criteria correlating to a depth of field between 0.1mm and 15mm, or a depth of field between 0.1mm and 1.0mm, for example a depth of field of approximately 0.5mm. The system can comprise spatial resolution criteria correlating to a depth of field of between 1.5mm and 10mm, for example a depth of field of approximately 7mm.

The first optical assembly can comprise a flange constructed and arranged to geometrically center the fiber.

The multiple tissue surface areas can comprise multiple tissue surfaces, each comprising an area between 0.1mm² and 20mm², or an area between 0.5mm² and 1.5mm², for example an area of approximately 1mm².

The multiple tissue surface areas can comprise multiple tissue surfaces, each comprising an equivalent diameter between 0.5mm and 1.5mm.

The multiple tissue surface areas can comprise multiple tissue surfaces, each comprising a major axis comprising a length between 0.5mm and 1.5mm.

The multiple tissue surface areas can comprise multiple tissue surfaces, each comprising a relatively circular geometry, or a relatively rectangular geometry. The multiple tissue surface areas can comprise multiple relatively flat tissue surfaces, or can comprise multiple peaks and valleys. The multiple tissue surface areas can comprise multiple tubular tissue surface areas, for example a segment of the esophagus.

The fiber can comprise a material selected from the group consisting of: zinc selenide, germanium; germanium oxide, silver halide; chalcogenide; a hollow core fiber material; and combinations of these.

The fiber can comprise a material relatively transmissive to wavelengths between 6µm and 15µm, or wavelengths between 8µm and 11µm.

The fiber can comprise a bundle of fibers. The bundle of fibers can comprise coherent or non-coherent fibers.

The fiber can comprise at least one anti-reflective coating. For example, the at least one anti-reflective coating can be positioned on at least one of the fiber proximal end or the fiber distal end. The at least one anti-reflective coating can comprise a coating selected from the group consisting of: a broadband anti-reflective coating such as a coating covering a range of 6µm - 15µm or a range of 8µm - 11µm; a narrow band anti-reflective coating such as a coating covering a range of 7.5µm - 8 µm or a range of 8µm - 9µm; a single line anti-reflective coating such as a coating designed to optimally reflect a single wavelength or a very narrow range of wavelengths in the infrared region; and combinations of these.

The fiber can further comprise a core comprising a diameter between 6µm and 100µm, or a diameter between 200µm and 400µm.

The fiber can further comprise a core and a surrounding cladding. The fiber further can comprise a core and an air envelope surrounding the core.

The fiber further can comprise a twist resisting structure surrounding at least a portion of the fiber between the fiber proximal end and the fiber distal end, for example a structure selected from the group consisting of: coil; braid; and combinations of these. The twist resisting structure can comprise a torque shaft. The torque shaft can comprise multiple layers of wires wound in opposite directions. The torque shaft can comprise four to twelve wires.

The system can be constructed and arranged to perform a manipulation on the fiber selected from the group consisting of: rotating the fiber; translating the fiber; and combinations of these. The fiber can comprise a service loop constructed and arranged to accommodate translation motion.

The fiber can further comprise a sleeve surrounding at least a portion of the fiber where the sleeve can comprise a material constructed and arranged to be non-reactive with at least a portion of the fiber. For example, the fiber can comprise a core, and the sleeve material can be constructed and arranged to be non-reactive with the core.

The sensor can comprise an infrared light detector. The sensor can comprise a sensor selected from the group consisting of: a photoconductor such as a mercury cadmium telluride photodetector or a mercury zinc telluride photodetector, a microbolometer; a pyroelectric detector such as a lithium tantalite detector or triclycine sulfate detector, a thermopile; and combinations of these.

The sensor can comprise a response time less than or equal to 200 milliseconds, or a response time less than or equal to 1 millisecond.

The sensor can comprise a cooling assembly constructed and arranged to cool one or more portions of the sensor. The cooling assembly can comprise a cooling assembly selected from the group consisting of: liquid nitrogen filled dewar; thermoelectric cooler; Stirling cycle cooler; and combinations of these.

The signal can comprise a voltage signal and/or a current signal. The signal can represent a change in received infrared light.

The system can further comprise a shaft, where the fiber is slidingly received by the shaft. The shaft can comprise a rounded tip. The shaft can comprise a material selected from the group consisting of: polyethylene; polyimide; polyurethane; polyether block amide; and combinations of these. The shaft can comprise a braided shaft. The shaft can be constructed and arranged for over-the-wire insertion into a body lumen. The shaft can be constructed and arranged for insertion into a nostril. The shaft can be constructed and arranged to be inserted through anatomy with a radius of curvature less than or equal to 4 inches, or a radius of curvature less than or equal to 2 inches, or a radius of curvature less than or equal to 1 inch.

The system can further comprise a second optical assembly constructed and arranged to receive infrared light from the fiber and direct light onto a receiving surface of the sensor. The second optical assembly can comprise an adjustment assembly constructed and arranged to allow at least two-dimensional positioning of the second optical assembly relative to the sensor.

The second optical assembly can comprise an optical element selected from the group consisting of: optical fiber; lens; mirror; filter; prism; amplifier; refractor; splitter; polarizer; aperture; and combinations of these. The second optical assembly can comprise an optical element constructed and arranged to perform an action on the received infrared light selected from the group consisting of: focus; split; filter; transmit without filtering; amplify; refract; reflect; polarize; and combinations of these.

The system can comprise a cooled housing and, at least a portion of the second optical assembly can be maintained within the cooled housing, for example a Stirling cooled housing.

The second optical assembly comprises a component comprising an anti-reflective surface.

The second optical assembly can comprise a component relatively transmissive of light with a wavelength between 6µm and 15µm, or a wavelength between 8µm and 11µm.

The second optical assembly can comprise a focusing lens. The focusing lens can be separated from a least a portion of the sensor by a gap, for example an operator adjustable gap.

The second optical assembly can comprise a filter. The filter can be relatively non-transmissive of light with a wavelength below 8µm and/or relatively non-transmissive of light with a wavelength above 11µm.

The second optical assembly can comprise a cold aperture.

The second optical assembly can comprise an immersion lens.

The second optical assembly can be constructed and arranged to overfill the receiving surface of the sensor with the infrared light received from the fiber. For example, the second optical assembly can be constructed and arranged to overfill the sensor to perform an action selected from the group consisting of: minimizing infrared light emanating from surfaces other than the fiber proximal end onto the sensor receiving surface; minimizing errors caused by light emanating from the fiber proximal end moving at least one of on or off the receiving surface; and combinations of these. The second optical assembly can be constructed and arranged to underfill the receiving surface of the sensor with the infrared light received from the fiber. For example, the second optical assembly can be constructed and arranged to underfill the sensor to maximize the amount of light received by the receiving surface of the sensor that emanates from the fiber proximal end. The system can be constructed and arranged to allow an operator to adjust the amount of at least one of overfill or underfill.

The second optical assembly can be constructed and arranged to deliver the infrared light received from the fiber in a pattern matching the geometry of the receiving surface of the sensor. In some embodiments, the system can be constructed and arranged to deliver the infrared light received from the fiber in a rectangular pattern to the receiving surface of the sensor, where the receiving surface of the sensor comprises a rectangular pattern. In some embodiments, the system can be constructed and arranged to deliver the infrared light received from the fiber in a circular pattern to the receiving surface of the sensor, where the receiving surface of the sensor comprises a circular pattern. In some embodiments, the system can be constructed and arranged to deliver the infrared light received from the fiber in an elliptical pattern to the receiving surface of the sensor, where the receiving surface of the sensor comprises an elliptical pattern. In some embodiments, the system can be constructed and arranged to deliver the infrared light received from the fiber in a square pattern to the receiving surface of the sensor, where the receiving surface of the sensor comprises a square pattern.

The system can further comprise a rotating assembly. The rotating assembly can be constructed and arranged to rotate the fiber and/or the first optical assembly. The system can further comprise a translating assembly constructed and arranged to translate the fiber. The system can be constructed and arranged to simultaneously rotate and translate the fiber or sequentially rotate and translate the fiber. The rotating assembly can be constructed and arranged to provide a 360° rotation. The rotating assembly can be constructed and arranged to provide a reciprocating rotation less than 360°, such as a reciprocating rotation between 45° and 320°, or a reciprocating motion of less than or equal to 180°, or a reciprocating motion of less than or equal to 90°.

The rotating assembly can comprise a rotational encoder.

The rotating assembly can be constructed and arranged to rotate the fiber at a velocity between 1000rpm and 15000rpm, or a velocity between 4000rpm and 8000rpm, for example a velocity of approximately 7260rpm.

The rotating assembly can comprise an adjustment assembly constructed and arranged to allow an operator to adjust the position of at least a portion of the fiber, for example the fiber proximal end. The adjustment assembly can be constructed and arranged to provide at least two dimensions of adjustment.

The system can further comprise a translating assembly constructed and arranged to translate the fiber and/or the sensor. The translating assembly can be constructed and arranged to translate the fiber in a reciprocating motion. The system can further comprise a rotating assembly constructed and arranged to rotate the fiber. The translating assembly can be further constructed and arranged to translate the rotating assembly. The system can be constructed and arranged to simultaneously rotate and translate the fiber, or sequentially rotate and translate the fiber.

The translating assembly can be constructed and arranged to translate the fiber a distance between 5mm and 100mm, or a distance between 10mm and 40mm, for example a distance of approximately 25mm.

The translating assembly can comprise a linear encoder. The translating assembly can comprise a yankee screw.

The translating assembly can be constructed and arranged to provide a relatively continuous translation of the fiber. The translating assembly can be constructed and arranged to translate the fiber for a first time period and a second time period, where the first and second time periods are separated by a delay.

The system can further comprise a user interface. The user interface can be constructed and arranged to display a graphical temperature map of the average temperature of each of the multiple tissue surface areas. The user interface can be constructed and arranged to depict temperature differences by varying a graphical parameter selected form the group consisting of: color; hue; contrast; and combinations of these. The user interface can be constructed and arranged to allow an operator to adjust a temperature versus a graphic parameter correlation.

The user interface can be constructed and arranged to display a temperature map of a two dimensional representation of body tissue and/or a three dimensional representation of body tissue.

The user interface can be constructed and arranged to display an alphanumeric table of temperature information.

The user interface can be constructed and arranged to display and continually update a temperature map of the average temperature of each of the multiple tissue surface areas. For example, the user interface can be constructed and arranged to update the temperature map every 0.1 seconds to every 30 seconds, or every 0.2 seconds to every 5 seconds, or every 0.5 seconds to every 2 seconds, for example approximately every 1 second.

The user interface can further comprise a user input component. The user input component can comprise a component selected from the group consisting of: touch screen monitor; a keyboard; a mouse; a joystick; and combinations of these.

The user interface can be constructed and arranged to allow an operator to calibrate the sensor. The user interface can be constructed and arranged to allow an operator to adjust a motion parameter selected form the group consisting of: a rotation parameter such as rotational travel distance and/or rotational speed; a translation parameter such as translation travel distance and/or translational speed; scanning pattern geometry; and combinations of these.

The user interface can be constructed and arranged to display other temperature information, for example at least one of peak temperature information and average temperature information for multiple tissue surfaces.

The system can further comprise a signal processing unit. The signal processing unit can be constructed and arranged to correlate the sensor signal into a table of temperature values correlating to the multiple tissue surface areas. The system can further comprise a video monitor, and the signal processing unit can produce a video signal constructed and arranged to drive the video monitor. The signal processing unit can comprise an algorithm, for example an algorithm constructed and arranged to perform a function selected from the group consisting of: averaging one or more values such as temperature values; finding the peak value of one or more temperature values; comparing peak values of one or more tissue areas; rate of change of tissue temperature; rate of rate of change of tissue temperature; determining an outlier value; and combinations of these. Additionally, the algorithm can be constructed and arranged to determine an area of tissue whose average temperature is higher than other tissue areas measured.

The system can further comprise at least one band, where the first optical assembly can collect infrared light emanating from the at least one band. The at least one band can comprise a proximal band, and the first optical assembly can be constructed and arranged to translate between a proximal position and distal position, and where the proximal band is positioned relative the proximal position. The at least one band can comprise a distal band, and the first optical assembly can be constructed and arranged to translate between a proximal position and distal position, where the distal band is positioned relative the distal position. The at least one band can comprise a distal band and a proximal band, and the first optical assembly can be constructed and arranged to translate between the distal band and the proximal band. The at least one band can comprise a material selected from the group consisting of: a thermally conductive material; aluminum, titanium, gold, copper, steel; and combinations of these. The at least one band can be constructed and arranged to cause the sensor to produce a predetermined signal when the first optical element receives infrared light from the at least one band.

The system can further comprise at least one temperature sensor constructed and arranged to measure a temperature of the at least one band. The at least one temperature sensor can comprise a sensor selected from the group consisting of: thermocouple; thermisters; and combinations of these. The system can be constructed and arranged to calibrate the sensor based on the measured temperature. The system can be constructed and arranged to calibrate the sensor multiple times, where the calibration can be based on the measured temperature. For example, the optical assembly can be constructed and arranged to translate, and the system can be constructed and arranged to calibrate the sensor for every optical assembly translation.

The at least one band can comprise a first band and a second band, and the system can further comprise a second temperature sensor constructed and arranged to measure a temperature of the second band. For example, the optical assembly can be constructed and arranged to translate, and the system can be constructed and arranged to calibrate the sensor two times for every optical assembly translation.

The at least one band can comprise a visualization marker, for example a marker selected from the group consisting of: a radiopaque marker such as a radiopaque marker band; an ultrasonically reflective marker; a visible light marker; a magnetic marker; and combinations of these.

The system can further comprise a positioning member. The positioning member can be constructed and arranged to position the first optical assembly at a distance from the tissue surface. The positioning member can be constructed and arranged to center the first optical assembly in a body lumen, for example in an esophagus.

According to another aspect, a system for producing surface temperature estimations of a tissue surface comprises an elongate probe comprising a first optical assembly constructed and arranged to receive infrared light emitted from multiple tissue surface areas and a fiber comprising a proximal end and a distal end, where the distal end is optically coupled to receive infrared light from the first optical assembly; and a sensor optically coupled to the fiber proximal end, where the sensor is constructed and arranged to produce a signal that correlates to an average temperature of each of the multiple tissue surface areas.

According to another aspect, a system for producing surface temperature estimations of a tissue surface comprises a fiber comprising a proximal end and a distal end, where the fiber is constructed and arranged to allow infrared light to pass therethrough; an optical assembly optically coupled to the distal end of the fiber, where the optical assembly is constructed and arranged to receive infrared light emitted from at least one tissue surface area; and a sensor optically coupled to the fiber proximal end, where the sensor is constructed and arranged to produce a signal based on the infrared light emitted from the at least one tissue surface area, and where the signal correlates to an average temperature of the at least one tissue surface area.

According to another aspect, a method of producing surface temperature estimations of a tissue surface comprises: selecting a system of as described herein; deploying at least a portion of the system to a tissue surface of a patient location; and producing surface temperature estimations in the region of the tissue surface.

### BRIEF DESCRIPTION OF THE DRAWINGS

The accompanying drawings, which are incorporated in and constitute a part of this specification, illustrate various embodiments of the present inventive concepts, and together with the description, serve to explain the principles of the inventive concepts. In the drawings:
**Fig. 1** is a schematic view of a temperature mapping system including a temperature measurement probe, consistent with the present inventive concepts.
**Fig. 2** is a sectional side view of the distal portion of the temperature measurement probe of Fig. 1 positioned in a body lumen, consistent with the present inventive concepts.
**Fig. 2A** is a magnified sectional side view of the distal portion of the temperature measurement probe of Fig. 2, including an infrared light collector, consistent with the present inventive concepts.
**Fig. 2B** is a perspective view of a component of the infrared light collector of Fig. 2A, including the pathway of collected infrared light, consistent with the present inventive concepts.
**Fig. 3** is an optical schematic of a "close-optimized" optical system, including cross sectional representations of tissue surface areas, consistent with the present inventive concepts.
**Fig. 4** is an optical schematic of a "range-optimized" optical system, including cross sectional representations of tissue surface areas, consistent with the present inventive concepts.
**Fig. 5A** is a perspective view of a sensor assembly and a rotating assembly, consistent with the present inventive concepts.
**Fig. 5B** is a perspective cross sectional view of the rotating assembly of Fig. 5A, consistent with the present inventive concepts.
**Fig. 6** is a perspective view of a translating assembly, consistent with the present inventive concepts.
**Fig. 7** is an optical schematic of an optical pathway proximate a sensor assembly, consistent with the present inventive concepts.
**Fig. 8A** is an optical schematic of an infrared detector illustrating projections of infrared light focused toward the detector, in a configuration that overfills the detector, consistent with the present inventive concepts.
**Fig. 8B** is an optical schematic of an infrared detector illustrating projections of the infrared light focused toward the detector, in a configuration that underfills the detector, consistent with the present inventive concepts.

### DETAILED DESCRIPTION OF THE DRAWINGS

Reference will now be made in detail to the present embodiments of the inventive concepts, examples of which are illustrated in the accompanying drawings. Wherever possible, the same reference numbers will be used throughout the drawings to refer to the same or like parts.

The terminology used herein is for the purpose of describing particular embodiments and is not intended to be limiting of the inventive concepts. As used herein, the singular forms "a," "an" and "the" are intended to include the plural forms as well, unless the context clearly indicates otherwise.

It will be further understood that the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") when used herein, specify the presence of stated features, integers, steps, operations, elements, and/or components, but do not preclude the presence or addition of one or more other features, integers, steps, operations, elements, components, and/or groups thereof.

It will be understood that, although the terms first, second, third etc. may be used herein to describe various limitations, elements, components, regions, layers and/or sections, these limitations, elements, components, regions, layers and/or sections should not be limited by these terms. These terms are only used to distinguish one limitation, element, component, region, layer or section from another limitation, element, component, region, layer or section. Thus, a first limitation, element, component, region, layer or section discussed below could be termed a second limitation, element, component, region, layer or section without departing from the teachings of the present application.

It will be further understood that when an element is referred to as being "on", "attached", "connected" or "coupled" to another element, it can be directly on or above, or connected or coupled to, the other element or intervening elements can be present. In contrast, when an element is referred to as being "directly on", "directly attached", "directly connected" or "directly coupled" to another element, there are no intervening elements present. Other words used to describe the relationship between elements should be interpreted in a like fashion (e.g., "between" versus "directly between," "adjacent" versus "directly adjacent," etc.).

Spatially relative terms, such as "beneath," "below," "lower," "above," "upper" and the like may be used to describe an element and/or feature's relationship to another element(s) and/or feature(s) as, for example, illustrated in the figures. It will be understood that the spatially relative terms are intended to encompass different orientations of the device in use and/or operation in addition to the orientation depicted in the figures. For example, if the device in a figure is turned over, elements described as "below" and/or "beneath" other elements or features would then be oriented "above" the other elements or features. The device can be otherwise oriented (e.g., rotated 90 degrees or at other orientations) and the spatially relative descriptors used herein interpreted accordingly.

The term "and/or" where used herein is to be taken as specific disclosure of each of the two specified features or components with or without the other. For example "A and/or B" is to be taken as specific disclosure of each of (i) A, (ii) B and (iii) A and B, just as if each is set out individually herein.

Provided herein is a temperature measurement system for producing a temperature map for multiple locations, such as a two or three dimensional surface of a patient's tissue. The system can include one or more sensors, such as infrared (IR) light detectors or other infrared sensors. The system can include a reusable portion, and one or more disposable portions. The system can include a probe, such as a probe constructed and arranged to be inserted into a body lumen such as the esophagus or colon. The probe can include an elongate member such as a shaft, and the system can be constructed and arranged to measure temperature at multiple tissue locations positioned at the side of the elongate member and/or forward of the distal end of the elongate member. The system or probe can be constructed and arranged as described in applicant's co-pending International Patent Application Serial Number PCT/US2011/061802, entitled "Ablation and Temperature Measurement Devices", and filed November 22, 2011, the contents of which is incorporated by reference in its entirety.

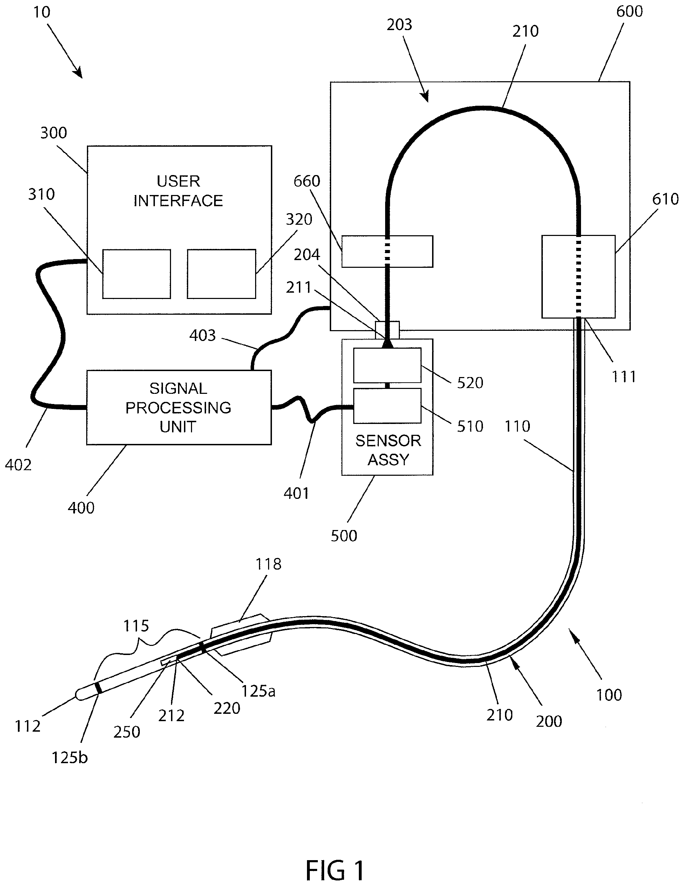

**Referring now to** **Fig. 1****,** a schematic view of a temperature mapping system including a temperature measurement probe is illustrated, consistent with the present inventive concepts. System 10 includes probe 100, sensor assembly 500, signal processing unit (SPU) 400, and user interface 300. Probe 100 includes shaft 110 which slidingly receives an elongate filament, fiber assembly 200. Fiber assembly 200 is constructed and arranged to collect at least infrared light emanating from one or more surface locations (e.g. one or more tissue surface locations) positioned radially out from the central axis of the distal portion of shaft 110. The collected infrared light travels proximally within fiber assembly 200 and is received by sensor assembly 500. Sensor assembly 500 converts the received infrared light to one or more information signals that are transmitted to SPU 400. System 10 can include motion transfer assembly 600, configured to cause fiber assembly 200 to translate and/or rotate, such as to collect infrared light from a series of tissue locations (e.g. a contiguous or discontiguous surface of tissue). SPU 400 converts the one or more information signals received from sensor assembly 500 into a series of temperature measurements that can be correlated to the series of tissue locations, such as to provide information regarding temperatures (e.g. average temperatures) present on a two and/or three dimensional tissue surface.

Shaft 110 includes proximal end 111 and distal end 112. Distal end 112 can comprise a rounded tip configured as shown for atraumatic insertion of probe 100 into a body lumen of a patient. Shaft 110 can comprise a material selected from the group consisting of: polyethylene; polyimide; polyurethane; polyether block amide; and combinations of these. Shaft 110 can comprise a braided shaft and/or include one or more braided portions constructed and arranged to provide increased column strength and/or improve response to a torque applied at or near proximal end 111 of shaft 110. Probe 100 can be configured for insertion over a guidewire, not shown but typically where shaft 110 includes a guidewire lumen or distal guidewire sidecar as is known to those of skill in the art. The distal portion of shaft 110 includes a relatively infrared transparent tube (i.e. an infrared transmissive tube), window 115, comprising a tubular segment which can include at least a portion which is transparent or relatively transparent to infrared light. Window 115 can comprise a material selected from the group consisting of: polyethylene such as high density polyethylene (HDPE) or low density polyethylene (LDPE); germanium or similarly infrared transparent materials; and combinations of these. In embodiments where shaft 110 includes a braid or other reinforcing structure, window 115 or a portion of window 115 can be void of the reinforcing structure.

Shaft 110 can be rigid, flexible, or include both rigid and flexible segments along its length. Fiber assembly 200 can be rigid, flexible, or include both rigid and flexible segments along its length. Shaft 110 and fiber assembly 200 can be constructed to be positioned in a straight or curvilinear geometry, such as a curvilinear geometry including one or more bends with radii less than or equal to 4 inches, less than or equal to 2 inches, or less than or equal to 1 inch, such as to allow insertion into the esophagus via a nasal passageway. In some embodiments, shaft 110 and fiber assembly 200 comprise sufficient flexibility along one or more portions of their length to allow insertion of probe 100 into a body lumen or other body location, such as into the esophagus via the mouth or a nostril, or into the lower gastrointestinal tract via the anus, and/or into the urethra. Shaft 110 can comprise an outer diameter less than 15Fr, such as a shaft with a diameter less than 12Fr, less than 9Fr, or less than 6Fr.

Fiber assembly 200 includes fiber 210 comprising proximal end 211 and distal end 212. Connector 204 is positioned on proximal end 211 and configured to mechanically and optically connect fiber assembly 200 to sensor assembly 500. In some embodiments, connector 204 comprises a linearly adjustable table or a two-dimensionally adjustable (X-Y) table constructed and arranged to allow precise positioning of fiber 210 relative to one or more components of sensor assembly 500. In some embodiments, one or two dimensional positioning can be performed by the manufacturer only. Fiber 210 can comprise one or more materials highly transparent to one or more ranges of infrared light wavelengths, such as one or more fibers comprising a material selected from the group consisting of: zinc selenide; germanium; germanium oxide; silver halide; chalcogenide; a hollow core fiber material; and combinations of these. Fiber 210 can be configured to be highly transparent to infrared light with wavelengths between 6µm to 15µm, or between 8µm and 11µm. In some embodiments, fiber 210 comprises multiple fibers, such as multiple fibers in a coherent or non-coherent bundle.

In some embodiments, proximal end 211 and/or distal end 212 of fiber 210 comprises a surface with a coating, such as an anti-reflective (AR) coating. System 10 can include one or more components that include an optical surface that receives infrared light and/or from which infrared light is emitted. These optical surfaces can include one or more anti-reflective coatings, such as a coating selected from the group consisting of: a broadband anti-reflective coating such as a coating covering a range of 6µm - 15µm or a range of 8µm - 11µm; a narrow band anti-reflective coating such as a coating covering a range of 7.5µm - 8 µm or a range of 8µm - 9µm; a single line anti-reflective coating such as a coating designed to optimally reflect a single wavelength or a very narrow range of wavelengths in the infrared region; and combinations of these. Anti-reflective coatings can be included to improve transmission by up to 30% per surface by reducing Fresnel losses at each surface. Anti-reflective coatings can be constructed and arranged to accept a small or large range of input angles.

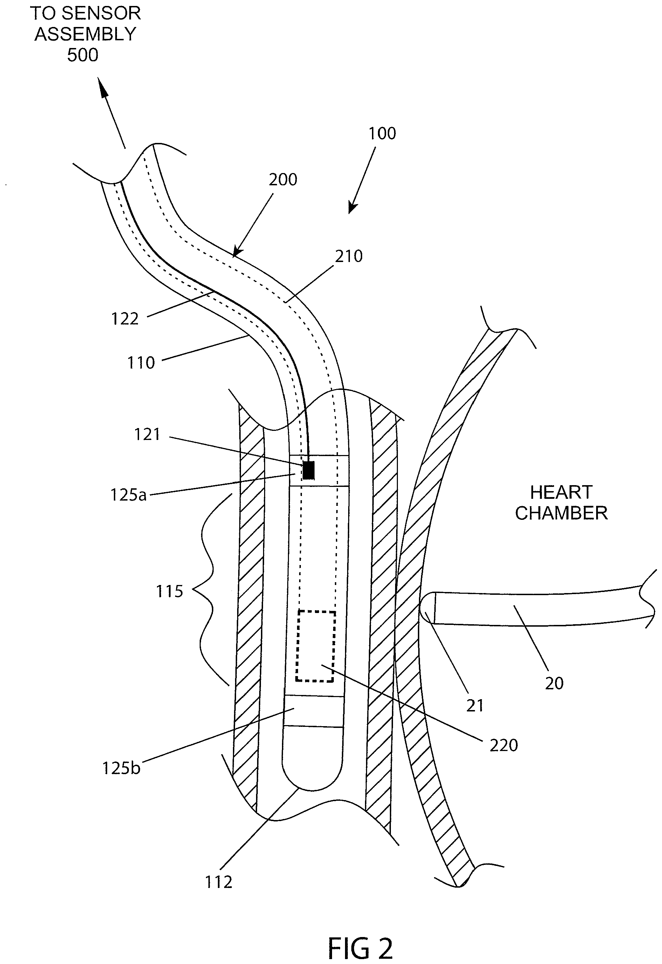

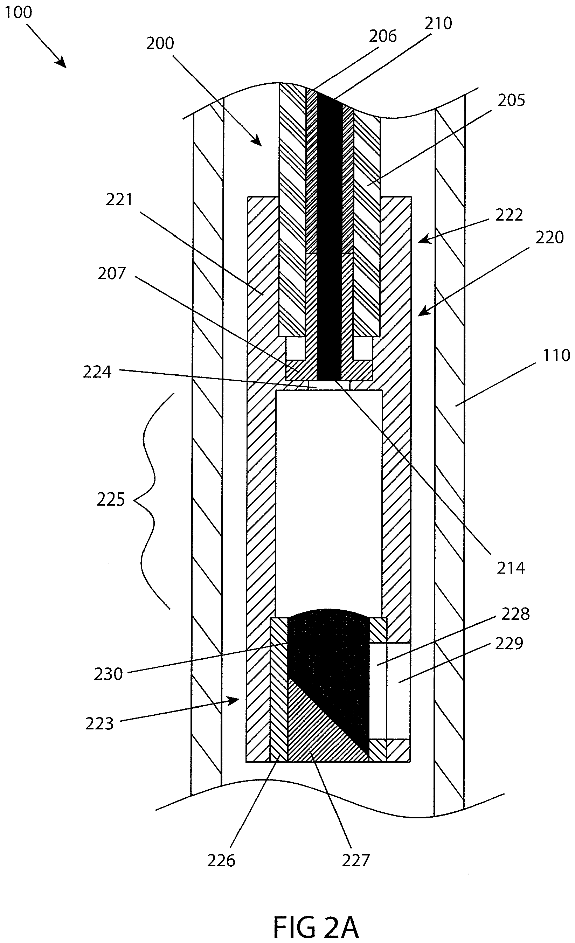

In some embodiments, fiber assembly 200 comprises a cladding, such as is described in reference to Fig. 2A herebelow. Cladding can be included to cause and/or maintain total internal reflection of the infrared light as it travels from the distal to proximal end of fiber assembly 200. Alternatively or additionally, fiber assembly 200 can comprise a coil, braid or other twist resisting structure surrounding optical fiber 210, such as to improve torsional response of fiber assembly 200. In some embodiments, fiber assembly 200 comprises a coil, braid or other surrounding element (e.g. a torque shaft) for improving torque response, such as is described in reference to Fig. 2A herebelow.

System 10 includes an optical assembly 250 comprising collector 220, which can be attached to distal end 212 of fiber 210. Collector 220 can include one or more optical components, such as one or more optical components used to perform an action on the collected infrared light, such as an action selected from the group consisting of: focus; split; filter; transmit without filtering (e.g. pass through); amplify; refract; reflect; polarize; and combinations of these. Collector 220 can include one or more optical components selected from the group consisting of: optical fiber; lens; mirror; filter; prism; amplifier; refractor; splitter; polarizer; aperture; and combinations of these. Collector 220 can include a housing and other mechanical, electrical and/or optical components, such as are described in reference to collector 220 of Fig. 2A herebelow. Collector 220 can comprise a finite rigid length, such as a rigid length less than 3cm, less than 2cm, less than 1cm or less than 0.5cm, such as to accommodate travel through a curvilinear path as described hereabove.

Infrared light which is emitted from a particular tissue location proximate to the distal portion of fiber assembly 200, and then passes through window 115, is collected by collector 220. Collector 220 is optically coupled to fiber 210 at distal end 212, such that the collected light travels proximally through fiber 210. Proximal end 211 is optically coupled to sensor assembly 500 such that the collected light is received by sensor assembly 500. A signal produced by sensor assembly 500 based on the collected light is correlated by SPU 400 to an estimated, average temperature, hereinafter "measured temperature", for that particular tissue location, hereinafter the "collection location". This measured temperature represents an average temperature of the entire surface of the collection location, which can include multiple different temperatures across its entire surface. In other words, the collected infrared light from each collection location travels proximally through fiber 210 as a single, undividable signal correlating to an average temperature of the entire collection location. Errors in the measured temperature can be caused by a factor selected from the group consisting of: unaccounted for and/or unknown infrared signal losses along system 10's optical pathway; unaccounted for and/or unknown infrared signal gains (e.g. an extraneous input of infrared light) along system 10's optical pathway; sensor assembly 500 inaccuracies or spurious signals; electrical signal noise; and combinations of these.

In some embodiments, collector 220 is constructed and arranged to collect light from a collection location (e.g. a tissue surface area) with an area of approximately 0.5mm² - 1,5mm², such as an area of approximately 1.0mm². In some embodiments, collector 220 is constructed and arranged to collect light from a relatively circular shaped area with a equivalent diameter ranging between 0.5mm and 1.5mm. In some embodiments, collector 220 is constructed and arranged to collect light from a rectangular or elliptical shaped area with a major axis between 0.5 and 1.5mm. Collection locations can comprise a broad range of sizes and shapes, such as locations comprising an area between 0.1mm² and 20mm². Collection locations can comprise various shapes such as a shape selected from the group consisting of: an ellipse such as a circle or an oval; a rectangle such as a square; a polygon such as a trapezoid; and combinations of these. The efficiency of collection of light from the collection location can vary over the collection area, for example the efficiency of collection from the center of the collection location can be greater than that from the periphery of the collection location, resulting in a weighting of the measured temperature towards that of the center of the collection location. System 10 can be constructed and arranged to collect light from multiple tissue surface areas, such as by rotating and/or spinning collector 220 as described in detail herein.

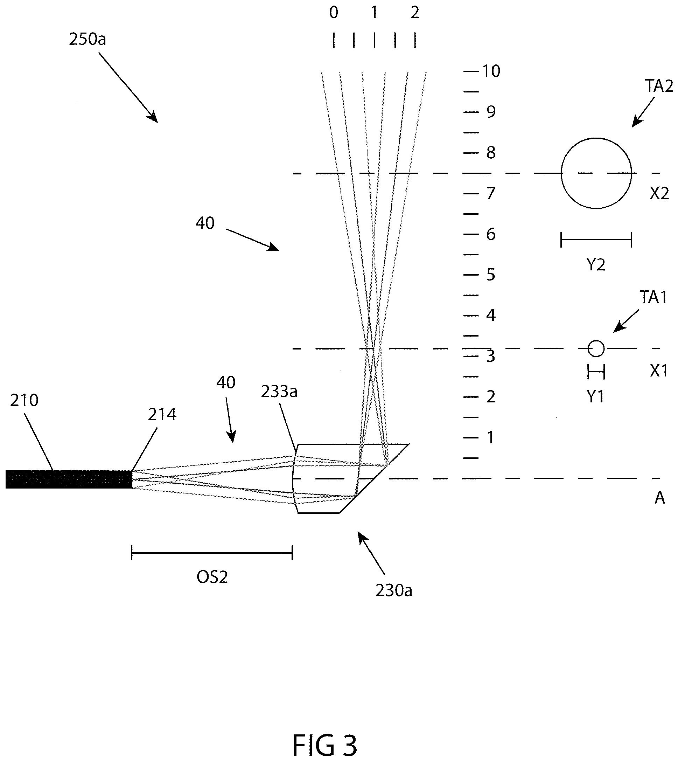

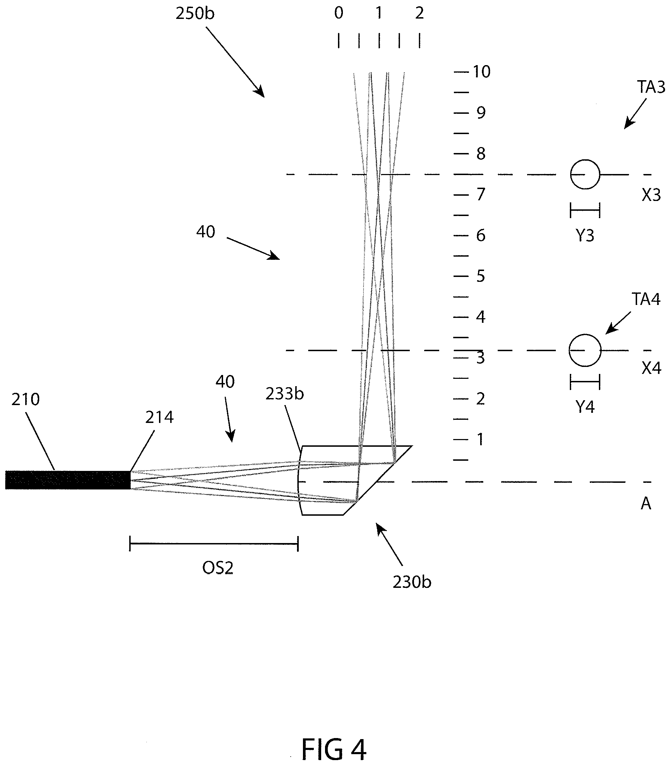

A collection location and/or groups of collection locations can comprise tissue that is relatively flat (e.g. the included tissue surface orthogonal to collector 220 has a relatively constant distance to collector 220), or it can comprise tissue that is undulating or otherwise includes peaks and/or valleys. System 10 can be configured to minimize temperature measurement errors by optics whose focus matches the topography of the tissue surface being measured. A non-limiting example of a system 10 optimized for tissue at a relatively uniform distance from probe 100 is described in reference to Fig. 3 herebelow. A non-limiting example of a system 10 optimized for tissue at a varying or unknown distance from probe 100 is described in reference to Fig. 4 herebelow.

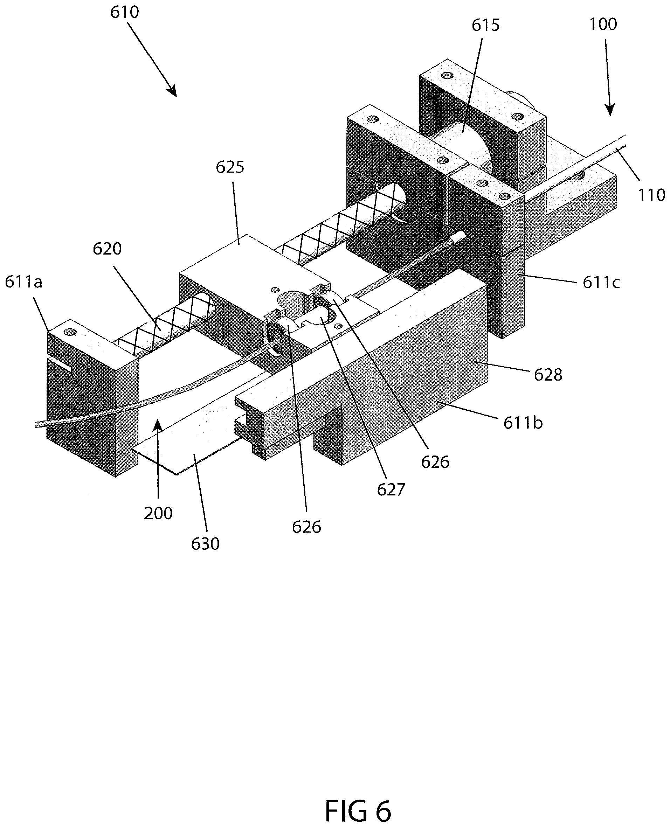

As described above, in some embodiments, fiber assembly 200, including collector 220, is configured to be translated and/or rotated, such as by translating assembly 610 and/or rotating assembly 660, respectively. Translating assembly 610 operably engages an axial segment of fiber assembly 200, and applies an axial force to cause fiber assembly 200 to move forward and back within shaft 110. Translating assembly 610 can be configured to create a reciprocating motion between 5mm and 100mm, such as between 10mm and 40mm, such as a reciprocating translation of approximately 25mm in each direction. In some embodiments, the magnitude of reciprocating motion is constructed and arranged to collect temperature information from a sufficient length of the esophagus during a cardiac ablation procedure. Fiber assembly 200 can comprise service loop 203, which comprises at least a flexible portion and is positioned and arranged to accommodate the translating motion without detaching from or otherwise imparting an undesired force to rotating assembly 660 and/or sensor assembly 500 (e.g. to accommodate the translating motion of fiber assembly 200). In some embodiments, translating assembly 610 includes one or more linear encoders or other position sensors constructed and arranged to produce a signal correlating to a linear position of fiber assembly 200. In some embodiments, translating assembly 610 is constructed and arranged as described in reference to Fig. 6 herebelow.

Rotating assembly 660 operably engages another axial segment of fiber assembly 200, and applies a rotational force to cause fiber assembly 200 and collector 220 to rotate, such as a continuous 360° rotation or a partial circumferential rotation (e.g. 45° to 320° reciprocating rotation). In an alternative embodiment, rotating assembly 660 is positioned distal to collector 220, distal position not shown but typically comprising a rotary motor positioned proximate distal end 212 and operably coupled to collector 220 such that at least a portion of collector 220 can be rotated without rotating fiber 210.

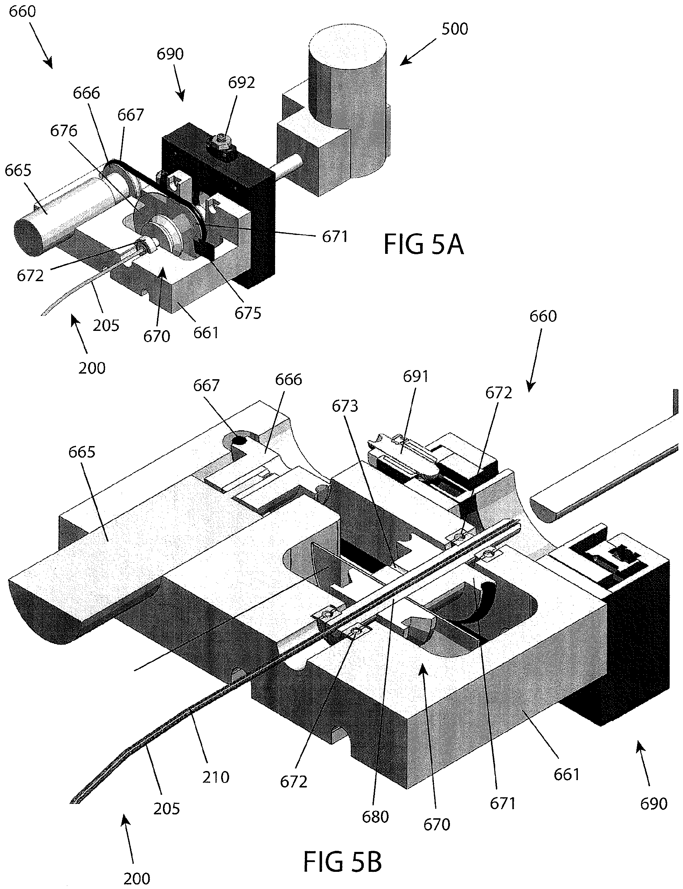

In some embodiments, rotating assembly 660 includes one or more rotary encoders or other position sensors constructed and arranged to produce a signal correlating to a rotational position of collector 220 and/or fiber assembly 200. In some embodiments, rotating assembly 660 is constructed and arranged as described in reference to Fig. 5A herebelow.

In some embodiments, rotating assembly 660 and/or sensor assembly 500, are positioned on or otherwise coupled to translating assembly 610, such that rotating assembly 660 and/or sensor assembly 500 translate along with fiber assembly 200. In these embodiments, service loop 203 can be avoided, such as to reduce the length of fiber assembly 200 and/or to reduce or eliminate any signal losses that occur during the flexing of service loop 203.

In some embodiments, translation and rotation occur simultaneously, such that the infrared light collected by collector 220 represents light collected from a helical pattern of collection locations. In other embodiments, a rotation (e.g. a 360° rotation of collector 220), is sequentially followed by a translation (e.g. an advancement or a retraction of collector 220), and the rotation-translation is repeated such that the infrared light collected represents a series of collection locations with a geometry comprising multiple two dimensional, parallel circles.

The information provided by sensor assembly 500 is used by SPU 400 to produce a table of collection location measured temperatures, which represent an estimated, averaged temperature for the collection location, as described above. The table provided by SPU 400 can be represented (e.g. by user interface 300) in the form of a temperature map correlating to the geometry of the multiple collection locations. In some embodiments, the multiple collection locations comprise a segment of tubular tissue, such as a segment of esophagus, and the temperature map is a two dimensional representation of the "unfolded" luminal wall or other body tissue. In other embodiments, a three dimensional representation of the luminal wall or other body tissue can be provided. The table or other representation can be updated on a regular basis, such as via data collected during a series of reciprocating translations in which collector 220 is continuously or semi-continuously rotated.

In some embodiments, a single forward or reverse translation over approximately 25mm occurs over a time period of between 0.1 seconds and 30 seconds, such as a time period between 0.2 seconds and 5.0 seconds, such as a time period between 0.5 seconds and 2.0 seconds, such as a time period of approximately 1.0 second. During the forward or reverse translation, collector 220 can be rotated, such as at a rotational velocity between 1000 rpm and 15000 rpm, or between 4000 rpm and 8000 rpm, such as approximately 7260 rpm. In some embodiments, a forward or reverse translation is separated by a reverse or forward translation, respectively, after a period of time. In other embodiments, a forward or reverse translation is initiated relatively immediately after the completion of the previous reverse or forward translation, respectively.

Sensor assembly 500 comprises one or more sensors configured to produce a signal based on the infrared light received from fiber assembly 200. As described above, the received infrared light can represent transmission of infrared light collected from a series of collection locations, as determined by translation and/or rotation of collector 220. SPU 400 can be configured to correlate the signals produced by sensor assembly 500 into a table of temperature values associated with a series of collection locations. Sensor assembly 500 can comprise a finite response time (e.g. a delay of output signal availability of one or more electronic components), during which a signal produced by sensor assembly 500, based on received infrared light, is unavailable (e.g. not accurate). In these embodiments, SPU 400 can be configured to discretely sample sensor assembly 500 to accommodate for any signal availability delay.

Sensor assembly 500 can include IR detector 510 such as an element selected from the group consisting of: a photoconductor such as a mercury cadmium telluride photodetector or a mercury zinc telluride photodetector; a microbolometer; a pyroelectric detector such as a lithium tantalite detector or triclycine sulfate detector; a thermopile; and combinations of these. In some embodiments, detector 510 comprises a response time less than 200 milliseconds, such as less than 1 millisecond.

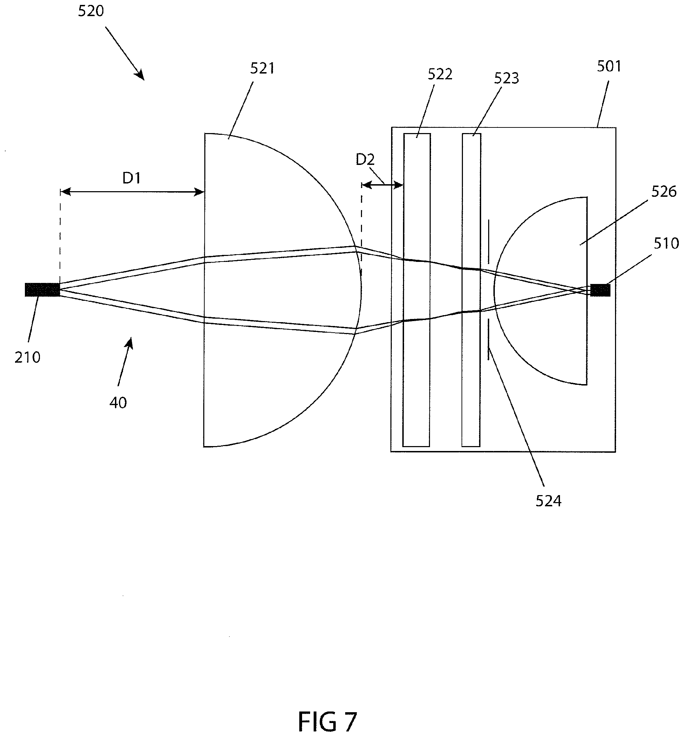

Sensor assembly 500 or another assembly of system 10 can include an optical assembly 520 comprising one or more optical components constructed and arranged to focus infrared light received from fiber assembly 200 onto IR detector 510. In some embodiments, optical assembly 520 is configured as described in reference to Fig. 7 herebelow.

IR detector 510 can be configured to convert the received infrared light into an electrical signal, such as a voltage and/or current signal correlating to the received infrared light. In some embodiments, IR detector 510 produces a differential signal, such as a voltage or current that correlates to a change in infrared light received, such as an infrared sensor manufactured by Infrared Associates of Stuart Florida, such as a sensor similar to Infrared Associates model number MCT-12-0.25SC. IR detector 510 can be configured with a broad spectral response and a high efficiency for converting infrared light into the electrical signal. In some embodiments, the sensitivity or other performance characteristic of IR detector 510 is related to the area of detector 510.

Sensor assembly 500 can comprise a cooling assembly, not shown but such as a liquid nitrogen filled dewar, a thermoelectric cooler, a Stirling cycle cooler, or another refrigeration and/or cooling assembly constructed and arranged to maintain one or more components of sensor assembly 500 at a temperature below room temperature, such as to improve the sensitivity, accuracy, noise characteristics or response time of sensor assembly 500.

SPU 400 receives electrical or other signals from sensor assembly 500 via a single or multi-conductor cable, conductor 401. Alternatively or additionally, SPU 400 can receive electrical or other signals from sensor assembly 500 via wireless communication means such as Bluetooth. SPU 400 includes mechanical components, electrical components (e.g. one or more microprocessors; memory storage devices; analog circuitry such as analog filters or amplifiers; digital circuitry such as digital logic; and the like) and/or software (e.g. software including one or more signal processing algorithms, software configured to drive user interface 300, and the like) sufficient to perform one or more signal processing tasks on the signals received from sensor assembly 500.

SPU 400 can be configured to produce a video signal which is transmitted to user interface 300 via a single or multiple conductor cable, conductor 402. Alternatively or additionally, SPU 400 can transmit a video signal to user interface 300 via wireless communication means such as Bluetooth.

User interface 300 includes monitor 310 which can comprise at least one touch-screen or other visual display monitor. User interface 300 can include input device 320, which can include a component configured to allow an operator of system 10 to enter commands or other information into system 10, such as an input device selected from the group consisting of: monitor 310 such as when monitor 310 is a touch screen monitor; a keyboard; a mouse; a joystick; and combinations of these.

In some embodiments, command signals provided by user interface 300, such as via input device 320, can be transmitted to SPU 400 via conductor 402. The command signals can be used to command and/or configure (e.g. calibrate) SPU 400, sensor assembly 500 (e.g. via conductor 401). In some embodiments, the command signals from user interface 300 are received by SPU 400 and transmitted to motion transfer assembly 600 via a single or multiple conductor cable, conductor 403. In these embodiments, one or more rotation and/or translation parameters can be adjusted by an operator of system 10, such as a parameter selected from the group consisting of: translation travel (e.g. axial distance); translation speed; rotational travel (e.g. portion of circumferential travel such as 360° or less than 360°); rotational speed; scanning pattern geometry; position or range of positions of collector 220 within window 115; and combinations of these.

As described above, SPU 400 can create a table of values correlating measured temperatures to one or more collection locations proximate window 115 of probe 100. The tabularized information can be represented in alphanumeric form on monitor 310 of user interface 300. Alternatively or additionally, the tabularized information can be represented in the form of a graphical temperature map correlating the series of tissue locations to a two-dimensional representation of the cumulative tissue location geometry. The graphical temperature map can correlate colors, hues, contrast and/or other graphical parameters to represent an array of temperatures. In some embodiments, the correlation between the temperature and the visualizable parameter is adjustable by an operator of the system, such as a temperature map including a range of colors wherein the color correlation can be adjusted (e.g. a threshold is adjusted to set a particular temperature to a color). In addition to displaying a temperature map, additional temperature information can be provided by SPU 400 and user interface 300, such as numeric values for peak temperature or an average temperature of the entire set of collection locations or for two or more subsets of collection locations such as operator definable subsets of collection locations.

SPU 400 can include one or more algorithms (e.g. programs stored in memory of SPU 400) used to process (e.g. mathematically process) the signals received from sensor assembly 500 or further process an already processed signal. In some embodiments, an algorithm is included to perform a function selected from the group consisting of: averaging one or more values such as temperature values; finding the peak value of one or more temperature values; comparing peak values of one or more tissue areas; rate of change of tissue temperature; rate of rate of change of tissue temperature; determining an outlier value; and combinations of these. In some embodiments, an algorithm is included to determine an area of tissue whose average temperature is higher than other areas measured.

In some embodiments, shaft 110 includes one or more functional elements, such as proximal band 125a and distal band 125b (generally band 125), which can be placed over and/or adjacent to the proximal and distal ends of window 115. Bands 125 can comprise a material selected from the group consisting of: a thermally conductive material; aluminum, titanium, gold, copper, steel; and combinations of these. Bands 125 can be constructed and arranged such that when collector 220 is positioned within a band 125 (e.g. collects infrared light transmitted from band 125), a signal is received by sensor assembly 500 comprising a pre-determined or otherwise separately measurable signal, such as a pre-determined pattern of infrared reflectance or emissivity, or a measurable temperature.

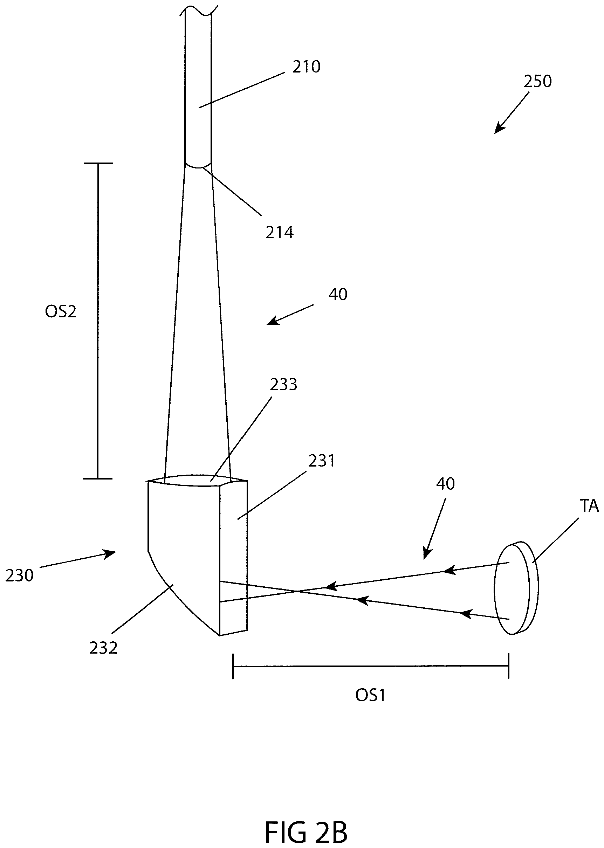

In some embodiments, one or more bands 125 comprise one or more temperature sensors, such as a thermocouple or a thermistor, not shown but such as temperature sensor 121 of Fig. 2 herebelow and connected to one or more electrical wires or other information transfer conduits which transmit the temperature sensor information to sensor assembly 500 and/or SPU 400. In these embodiments, the temperature reading received from a band 125 can be correlated to the infrared light collected at that location by collector 220, such as to perform a calibration procedure of system 10. In some embodiments, a calibration procedure is performed at least once for each set of forward and back reciprocating translations (e.g. when collector 220 is within proximal band 125a or within distal band 125b). In other embodiments, a calibration procedure is performed at least twice for each set of forward and back reciprocating translations (e.g. when collector 220 is within proximal band 125a and when collector 220 is within distal band 125b).

One or more bands 125 or another component of probe 100 can be configured as a visualization marker, such as a marker selected from the group consisting of: a radiopaque marker such as a radiopaque marker band; an ultrasonically reflective marker; a visible light marker; a magnetic marker; and combinations of these. Bands 125 or other visualization markers of probe 100 can be used by a clinician to advance, retract, rotate or otherwise position probe 100 in relation to a body structure such as placement using fluoroscopy or ultrasound to position window 115 proximate the heart when probe 100 is placed into the esophagus (as described in reference to Fig. 2 herebelow).

In some embodiments, probe 100 comprises an functional element constructed and arranged to position a distal portion of probe 100 (e.g. window 115) relative to tissue, such as positioning element 118, shown in a deployed, radially expanded state in Fig. 1. Positioning element 118 can be constructed and arranged to be radially expanded and/or radially contracted. In some embodiments, positioning element 118 is constructed and arranged to position probe 100 in a body lumen, such as a balloon, an expandable cage, an expandable stent, and/or radially deployable arms constructed and arranged to center window 115 in a body lumen, such as the esophagus. Positioning element 118 can be constructed and arranged to position one or more portions of probe 100 towards and/or away from tissue. In some embodiments, probe 100 and/or positioning element 118 is constructed and arranged as described in applicant's co-pending International Patent Application Serial Number PCT/US2011/061802, entitled "Ablation and Temperature Measurement Devices", filed November 22, 2011, the contents of which is incorporated by reference in its entirety.

**Referring now to** **Fig. 2****,** the distal end of the temperature measurement probe of Fig. 1 is illustrated, positioned in an esophagus and positioned near a heart chamber, consistent with the present inventive concepts. Probe 100 can be attached to one or more assemblies of system 10 described in reference to Fig. 1. Probe 100 includes shaft 110 and fiber assembly 200 including fiber 210 and an optical assembly comprising collector 220. Shaft 110 includes window 115 comprising one or more materials with high transmissivity to the desired wavelengths of infrared light. Positioned at each end of window 115 are proximal band 125a and distal band 125b (generally 125). Bands 125 can include one or more temperature sensors, such as one or more thermocouples, thermisters, or other temperature sensors. In the illustrated embodiment, thermocouple 121 is positioned on band 125a and is configured to measure temperature information of band 125a proximate one or more tissue T locations. Bands 125 can be positioned within a wall of shaft 110; on an outer surface of shaft 110 such as around an outer circumference of shaft 110, and/or on an inner surface of shaft 110 such as around an inner circumference of shaft 110. Bands 125 can comprise an infrared-opaque material and/or a material with a known emissivity, such that fiber assembly 200 records the infrared temperature information of bands 125 when infrared light emitted from a band 125 is received by collector 220. Bands 125 can comprise a radiopaque material such that bands 125 are visible to a visualization instrument so as to position distal end 112 of shaft 110, for example at a location within the esophagus most proximate a patient's heart. Examples of visualization instruments include: an MRI; a CT scanner; a fluoroscope or other x-ray instrument; and combinations of these.

Thermocouple 121 records temperature information, such as temperature dependent voltage information received by sensor assembly 500 and/or a signal processor, such as signal processor 400 of Fig. 1, via conduit 122, comprising one or more wires or other signal carrying conduits. Thermocouple 121 can be positioned within band 125a; on an outer surface of band 125a; on an inner surface of band 125a; and/or within a lumen of shaft 110. In some embodiments, thermocouple 121 is positioned within a lumen of shaft 110, and band 125a is positioned on an outer surface of shaft 110 such that band 125a surrounds shaft 110 and thermocouple 121.

In some embodiments, probe 100 can be used to monitor the temperature of the surface of the esophagus, such as during a clinical procedure where thermal application therapies (e.g. those using ablative heat or cold) are applied to the posterior wall of the heart. In some embodiments, probe 100 is inserted into the esophagus over a guidewire (e.g. over-the-wire insertion into a body lumen) and the guidewire is removed or partially withdrawn prior to performing one or more temperature measurements, such as to remove the guidewire from proximity to window 115. Thermal application therapies can include ablation therapies, such as RF ablation therapies performed using an ablation catheter, such as ablation catheter 20 comprising tip electrode 21. Thermal application therapies can also include but are not limited to therapies selected from the group consisting of: multiple electrode RF treatment; cryogenic treatment; laser energy treatment; ultrasound energy treatment; and combinations of these. In the embodiment of Fig. 2, probe 100 is shown positioned such that optical viewing window 115 (the space between bands 125) is relatively centered with respect to electrode 21 of ablation catheter 20. Bands 125 can be visualizable such as to aid in positioning probe 100 in such a manner, for example under fluoroscopy when bands 125 comprise at least a radiopaque portion.

**Referring now to** **Fig. 2A****,** a magnified sectional side view of the distal portion of the temperature measurement probe of Fig. 2 is illustrated, including an infrared light collector and consistent with the present inventive concepts. Probe 100 includes fiber assembly 200. Fiber assembly 200 includes fiber 210 and an optical assembly configured to collect infrared light, collector 220 positioned distal to fiber 210 as shown. Infrared light collected by collector 220 is focused onto distal face 214 of fiber 210. Fiber assembly 200 is configured to be rotated and/or translated within shaft 110, such as by rotating assembly 660 and/or translating assembly 610 described in reference to Fig. 1 hereabove. Optical fiber 210 can comprise a core diameter of between 6 and 1000 microns, such as a fiber with a diameter between 200 microns and 400 microns. Fiber 210 material can include a material configured to optimally transmit (e.g. provide minimal impedance to) infrared light in the 6-15 micron wavelength range, for example in the 8-11 micron wavelength range. In some embodiments, fiber 210 comprises a polycrystalline material such silver halide or one or more other materials with high transmissivity to a desired range of wavelengths of infrared light, such as a material selected from the group consisting of: zinc selenide; germanium; germanium oxide; chalcongenide; a hollow core material; and combinations of these. Optical fiber 210 can include a cladding layer which can be constructed and arranged to cause and/or maintain total internal reflection in the core of fiber 210 to ensure efficient transmission of the collected infrared light from the distal to proximal end of fiber 210. In some embodiments, fiber 210 does not include a cladding layer, instead an air envelope is positioned around the fiber to cause and/or maintain total internal reflection.

Fiber assembly 200 further includes sleeve 206, flange 207, torque shaft 205, and optical element 230. Sleeve 206, which surrounds the majority of the length of optical fiber 210, can be configured to protect optical fiber 210, for example by preventing direct contact between fiber 210 and torque shaft 205. Sleeve 206 can include infrared-opaque polymer tubing which can be configured to be non-reactive with fiber 210, for example when fiber 210 comprises a polycrystalline material. Probe 100 can include one or more components which contact fiber 210. These components can comprise a material configured to avoid damaging fiber 210, such as titanium, ceramic and/or polymer based components chosen to be non-reactive with a polycrystalline-based fiber 210. In some embodiments, another component of probe 100 can be polycrystalline-based, such as optical element 230, where its contacting components comprise a non-reactive material such as titanium, ceramic and/or a polymer.

In the embodiment of Fig. 2A, torque shaft 205 surrounds sleeve 206, flange 207 and optical fiber 210 along the length of fiber 210. Torque shaft 205 is configured to transmit rotational and translational forces from rotating and translating assemblies 660 and 610 respectively, from the proximal portion of fiber assembly 200, to collector 220 at the distal end of fiber assembly 200, such that fiber assembly 200, including collector 220 rotates and/or translates within shaft 110 as described herein. In some embodiments, torque shaft 205 comprises multiple wires or other filaments such as stainless steel or titanium wires. Shaft 205 can comprise multiple braided wires and/or multiple layers of wires wound in one or more directions (e.g. wound in opposite directions in two or more alternating layers). In some embodiments, up to 16 wires (e.g. 4 to 12 wires) are included in one or more layers of shaft 205.

Collector 220 can comprise structural and mechanical elements fabricated from a ceramic or titanium material, such as to prevent degradation of any polycrystalline-based components of collector 220, fiber 210 and/or another component of fiber assembly 200. Collector 220 includes proximal portion 222, including an opening, window 224. Collector 220 further includes distal portion 223, which includes an opening, window 229. Collector 220 includes a housing, housing 221 as shown. Torque shaft 205 and optical fiber 210 are attached to collector 220 at proximal portion 222. Flange 207 can surround the distal portion of fiber 210 and can include similar or dissimilar materials as sleeve 206. Flange 207 can be configured to geometrically center optical fiber 210 within window 224. In some embodiments, sleeve 206 and flange 207 can comprise a single component. A mid portion of collector 220 can include a gap, optical separation window 225, positioned between distal face 214 of fiber 210 and the opposing face of optical element 230. Optical separation window 225 facilitates focusing of infrared light from optical element 230 onto distal face 214 of optical fiber 210, as described in reference to Fig. 2B herebelow. Distal portion 223 of collector 220 houses optical element 230. Optical element 230 is surrounded by housing 226. Housing 226 can include a material similar or dissimilar to sleeve 206 and/or flange 207. Housing 226 comprises an opening, window 228, and can further comprise cap 227 configured to secure optical element 230 within housing 226, as well as rotationally align optical element 230 such as to be oriented towards window 228. Alternatively, optical element 230 can be fixed directly to distal portion 223 avoiding the need for housing 226.

Optical element 230 can include one or more components selected from the group consisting of: a lens; a mirror; a prism; and combinations of these. Optical element 230 can include similar or dissimilar materials to optical fiber 210. Optical element 230 can include one or more materials, such as a material configured to transmit (e.g. be relatively transparent to) infrared light and/or a material configured to reflect infrared light. In some embodiments, optical element 230 comprises an infrared transparent material attached to an infrared reflected material, such as is described in reference to optical element 230 of Fig. 2B herebelow.

**Referring additionally to** **Fig. 2B****,** a perspective view of a segment of probe 100 of Fig. 2A is illustrated, including the pathway of collected infrared light and consistent with the present inventive concepts. In Fig. 2B, fiber 210 and optical assembly 250, including optical element 230, are shown, with other components of probe 100 removed for illustrative clarity. Optical element 230 includes planar surface 231, angled surface 232, and convex surface 233. In some embodiments, planar surface 231 can comprise a convex or concave geometry. Probe 100 is configured to collect and focus IR light 40 emanating from a surface of tissue area, tissue area TA, onto distal face 214 of optical fiber 210. First optical separation distance OS1 comprises the distance between tissue area TA and planar surface 231 of optical element 230. Second optical separation distance OS2 comprises the distance between distal face 214 of optical fiber 210 and convex surface 233 of optical element 230. Distance OS2 is determined based on the focusing requirements of optical element 230 and desired optical resolution of optical assembly 250, and is maintained by the geometry of collector 220 (e.g. the geometry of window 225 shown in Fig. 2A).

In the embodiments of Figs. 2B, 3 and 4, fiber 210 and optical element 230 are constructed and arranged to define an optical assembly 250. In some embodiments, IR light 40 collected by optical assembly 250 from tissue area TA represents the infrared light collected from the conical projection of optical element 230 from planar surface 231 onto the surface of tissue area TA. IR light 40 collected from tissue area TA that is within the conical projection travels distance OS1 towards planar surface 231 of optical element 230. In other embodiments, a collimated or nearly collimated projection, a projection with a long beam-waist, and/or other geometric projection represents the collected infrared light. IR light 40 travels through optical element 230 towards angled surface 232, and is then reflected towards surface 233. IR light 40 is then focused by surface 233 onto the distal face 214 of optical fiber 210. Planar surface 231 can comprise a flat, convex, concave, curved, and/or an irregularly shaped surface configured to collect IR light 40 emitted from a surface of tissue area. Planar surface 231 can include a polished surface and/or it can include an anti-reflective coating as described above in reference to Fig. 1.

IR light 40 emitted from tissue area TA is collected by optical element 230 at surface 231, and travels through optical element 230 towards angled surface 232. Angled surface 232 can include a 45° angle, and can be coated, for example with a reflective coating such as a protected aluminum (PAL) or gold coating. Angled surface 232 can be configured to reflect IR light 40 perpendicularly towards convex surface 233 of optical element 230. In some embodiments, angled surface 232 can comprise an angle greater than or less than 45°, such as to meet optical requirements of optical assembly 250.

Infrared light reflected from angled surface 232 is reflected toward convex surface 233. Convex surface 233 is configured to focus IR light 40 onto the distal face 214 of optical fiber 210. Surface 233 can be coated with an anti-reflective coating, such as an anti-reflective coating similar or dissimilar to the anti-reflective coating of planar surface 231. In some embodiments, surface 231 can be flat, concave or include an irregularly shaped surface, such as to meet the optical requirements of optical assembly 250.

Optical assembly 250 comprises a numerical aperture comprising the range of angles over which infrared light is collected from a surface. Optical assembly 250's numerical aperture (NA) is the sine of, and therefore describes, the angle of the steepest light ray entering optical assembly 250 from tissue area TA and passing through fiber distal end 212. Since the NA is defined as the sine of this angle, the angle of the steepest ray increases as numerical aperture increases. The amount of IR light 40 collected from a particular point within a tissue area increases as the numerical aperture of optical assembly 250 increases. Generally, as the amount of IR light 40 collected increases (e.g. a higher NA), optical assembly 250 signal to noise ratio improves. Fiber 210 comprises an inherent maximum acceptance numerical aperture determined by the material of the core and cladding of fiber 210, specifically the index of refraction of these materials. IR light 40 entering fiber 210 at angles greater than the fiber 210's maximum numerical aperture will not be transmitted by fiber 210 to the sensor assembly 500. In some embodiments, fiber 210 comprises a maximum numerical aperture of 0.28 and a core diameter of 400 microns. In these embodiments, optical assembly 250 outputs a numerical aperture ranging from 0 to 0.28, for example, 0.11 to 0.14, thereby underfilling the maximum numerical aperture of the fiber.

An average temperature can be calculated for the tissue area TA based on the amount of IR light 40 which has been collected. In applications where this average temperature is to be displayed, or otherwise presented as a temperature versus two-dimensional location map (i.e. a map of multiple tissue locations), the area of each conical projection of optical assembly 250 is used to create this map and must be known or otherwise estimated. In some embodiments, distance OS1 to each measured tissue area TA can have minimal variance, and in other embodiments distance OS1 to each measured tissue area TA can have larger fluctuations. Provided herebelow in Fig. 3, optical assembly 250a is constructed and arranged to have a minimal depth of field, but higher optical assembly 250a Numerical Aperture and higher optical resolution for applications where the tissue surface distances are relatively uniform. Provided herebelow in Fig. 4, optical assembly 250b is constructed and arranged to have an extended depth of field, which correlates to a lower optical assembly 250b Numerical Aperture and lower optical resolution, such as for applications where the tissue surface distances are less uniform (greater variation in distances such as due to a non-uniform tissue surface).

**Referring now to** **Fig. 3****,** an optical schematic of a "close-optimized" optical system is illustrated, including cross sectional representations of tissue surface areas and consistent with the present inventive concepts. The close-optimized optical assembly 250a is optimized to have a higher optical assembly 250a Numerical Aperture and higher optical resolution resulting in a smaller depth of field. These close-optimized embodiments are useful when the multiple tissue surface locations to be measured are known or likely to be within a limited range of distances from central axis A of optical element 230a. This optimization can be used to improve the accuracy and spatial resolution (e.g. pixel resolution) of a map of temperature versus tissue location, as is described in detail in reference to Fig. 1.

Optical assembly 250a, including optical element 230a, is constructed and arranged to have a small focal length near optical element 230a and/or a window surrounding optical element 230a as has been described hereabove. This short focal length results in a relatively short depth of field. In some embodiments, the focal length (e.g. a distance measured from the center axis of optical element 230a) can range from 1 to 10mm, such as from 1mm to 5mm, such as approximately 3.2mm and the associated tissue area from which infrared light is collected can range from 0.5mm² to 1.5mm². In visible light cameras, depth of field correlates to a range of distances in which a produced image appears acceptably sharp. In the temperature measurement systems and devices of the present invention, depth of field correlates to a range of distances around, before or beyond the focal length in which the tissue area from which infrared light collected is within an acceptable range of cross sectional areas, such as a range selected to meet a useful and acceptable spatial resolution criteria for temperature data collection. Depth of field varies depending on optical component 230a configuration, the numerical aperture of fiber 210, and distance OS2. In some embodiments, the depth of field can range from 0.1 to 15.0mm, such as from 0.1mm to 1.0mm, such as a depth of field of approximately 0.5mm around or beyond the optimal focal length. Optical element 230a focuses the collected infrared light 40 onto distal face 214 of optical fiber 210, such that the collected light can travel proximally to one or more sensor devices as described herein.

In some applications, tissue is positioned on or near the outer surface of an infrared transparent tube surrounding optical element 230a, such as window 115 of Fig. 1. Tissue in close proximity to the surrounding tube is often encountered in applications where the catheter is inserted into a body lumen having a diameter smaller than or relatively equivalent to that of the tube, for example when the body lumen includes a colon; urethra; and combinations of these. In some embodiments, the body lumen can include the esophagus, for example when the system is used to monitor the temperature of the esophagus during cardiac ablation. The properties of the mammalian esophagus are such that the esophageal wall can collapse around the tube. In these embodiments, the focal length can be chosen to approximate the orthogonal distance between the central axis of optical element 230a and the outer surface of the surrounding tube, and the depth of field can be chosen to be small.

In optical assembly 250a, optical element 230a is configured, and optical separation distance (OS2) is selected, such that the focal length of optical assembly 250a is a distance X1 from the center axis A of optical element 230a. At the focal length X1 of optical assembly 250a, a cross sectional view of area TA1 is shown and includes diameter Y1. Diameter Y2 of area TA2 is determined by the cone of infrared light collected from tissue at distance X2 as shown, such that area TA2 is significantly larger than area TA1. Optical assembly 250a can comprise spatial resolution criteria (e.g. spatial accuracy criteria) that defines a corresponding depth of field centered about focal length X1. In some embodiments, distance X2 is within the focal length of optical assembly 250a, such that tissue positioned at distances between X1 and X2 are accurately measured. In other embodiments, distance X2 is outside the depth of field, and accurate temperature measurements must be performed within the appropriate depth of field (i.e. at a threshold distance less than X2). In the embodiment of Fig. 3, tissue locations within optical assembly 250's resolution-based depth of field can have a cross sectional area approximately equal to area TA1, such as an area within 0.01mm² of the area of TA1. For tissue locations positioned outside the depth of field, such as area TA2, as shown, the cross sectional area is larger than area TA1, such as comprising an area more than 1mm² greater than the area of TA1. In one embodiment of optical assembly 250a, fiber optic 210 has approximately a 400µm diameter core; distance OS2 comprises a length of approximately 4.5mm; optical element 230a is fabricated of zinc selenide; and lens surface 233a has a convex radius of approximately 3mm. In this particular embodiment, focal length X1 is approximately equal to 3.5mm, and TA1 has a diameter Y1 of approximately 0.4mm (e.g. area TA1 is approximately 0.13mm²). Optical assembly 250a can include spatial resolution criteria such that an acceptable depth of field includes tissue positioned at distances out to 7.5mm (i.e. tissue areas with a diameter greater than 1mm). Alternatively, optical assembly 250a can include spatial resolution criteria such that an acceptable depth of field includes tissue areas with a diameter of approximately 1mm (i.e. a depth of field that does not include tissue positioned at distance X2).

As described above, the close-optimized optical assembly 250a can be constructed and arranged to provide accurate temperature measurements for tissue areas at a relatively fixed distance from central axis A. Optical assembly 250a can include spatial resolution criteria (e.g. spatial accuracy criteria) that determines an acceptable depth of field from its focal length X1. Optical assembly 250a can be utilized if tissue to be measured would be primarily positioned near (e.g. close to or in contact with luminal wall tissue) the outer surface of an infrared transparent tube surrounding optical element 230a, such as window 115 of Fig. 1. Maximal resolution is achieved at tissue surfaces positioned at these close distances. Tissue positioned at greater distances, result in lower spatial accuracy.

**Referring now to** **Fig. 4****,** an optical schematic of a "range-optimized" optical system is illustrated, including cross sectional representations of tissue surface areas and consistent with the present inventive concepts. The range-optimized optical assembly 250b is optimized to provide accurate temperature measurements for tissue surfaces positioned at greater distances and/or greater variation in distances (e.g. from central axis A) than the close-optimized optical assembly 250a of Fig. 3. For example, optical assembly 250b can be constructed and arranged to provide a consistent resolution over a larger depth of field and/or provide a more accurate resolution at greater distances from the system's focal length. Optical assembly 250b can be selected for use when the multiple tissue surface locations to be measured are known or likely to be within a wider range of distances from central axis A of optical element 230b. This optimization can be used to improve the accuracy of a map of temperature versus tissue location, as is described in detail in reference to Fig. 1. The trade-off for the range-optimized optical assembly 250b of Fig. 4 is that the minimum temperature measurement area is not as small as that in the close-optimized optical assembly 250a of Fig. 3. In other words, optical assembly 250b results in a reduction in spatial resolution at the focal length, however its spatial resolution is reasonably consistent over a much larger range of distances from the focal length.

Optical assembly 250b including optical element 230b is constructed and arranged to have a focal length X3 and a relatively long depth of field. In some embodiments, the depth of field can range from 1.5mm and 10mm, such as a depth of field of approximately 7mm. Optical element 230b focuses the collected infrared light 40 onto distal face 214 of optical fiber 210, such that the collected light can travel proximally to one or more sensor devices as described herein.

In some applications, tissue is positioned away from, both close and away from and/or at unknown distances from an infrared transparent tube surrounding optical element 230b, such as window 115 of Fig. 1. Tissue positioned away and/or at varying distances from the surrounding tube can be encountered in larger body lumens such as the esophagus or the stomach. When positioned in a body lumen such as the esophagus, one or more distances between the tissue and the surrounding tube can be unknown. For these various applications, the focal length can be chosen to approximate the natural or relaxed radius of the body lumen, while the depth of field can be chosen to approximate the variation in the radius of the body lumen or variation in the position of the device within the body lumen (e.g. positioned in contact with a circumferential segment of a luminal wall while at a relatively large distance from an apposing circumferential segment of the wall). In some embodiments, the body lumen can include the esophagus, for example when the system is used to monitor the temperature of the esophagus during cardiac ablation and when the esophageal wall is assumed to be within a range of distance from the surrounding tube. For example, when positioned against one circumferential segment of the esophageal wall (e.g. 0 mm from the surrounding tube or approximately 1.5mm from center axis A of optical element 230b), the apposing segment of the esophageal wall can be from 0mm to 10mm from the surrounding tube. In these embodiments, the optimal focal length can be chosen to approximate half the distance between the outer surface of the surrounding tube and the greatest assumed distance to the tissue surface (e.g. a focal length between 0mm to 10mm). The depth of field can be configured to approximate the range of distances assumed or expected to be encountered during the performance of a temperature measurement.

In optical assembly 250b, optical element 230b is configured, and optical separation distance OS2 is selected such that the focal length of optical assembly 250b is a distance X3 from the center axis A, of the optical element 230b. At the focal length X3 of optical assembly 250b, the diameter of tissue area TA3 is represented by diameter Y3. In some embodiments distance X3 (i.e. the focal length) can range from 4mm to 10mm, such as approximately 7mm, and the diameter Y3 of tissue area TA3 can range from 0.5mm² to 1.5mm². A cross sectional view of the tissue area at a distance X4, area TA4, is shown in Fig. 4. Tissue locations within the depth of field of the optical assembly 250b have a cross sectional area approximately equal to area TA3, such as an area within 0.2 mm², within 0.1 mm², or within 0.01mm² of the area of TA3. Optical assembly 250b has a long depth of field, such that tissue locations positioned an appropriate distance away from the focal length (as determined by optical assembly 250b's spatial resolution criteria), such as distance X4 as shown (or at a distance nearer to a surrounding tube, not shown), comprises a cross sectional area approximately equal to area TA3, such as comprising an area within 0.2mm² of the area of TA3.

In one embodiment of optical assembly 250b, fiber optic 210 comprises an approximately 400µm diameter core; distance OS2 comprises a length of approximately 4.2mm; optical element 230b is comprised of zinc selenide; and lens surface 233b has a convex radius of approximately 4mm. In this particular embodiment, the focal length X3 would be approximately equal to 7.5mm, and TA3 would have a diameter Y3 of approximately 1.0mm (i.e. area TA3 comprises an area of approximately 0.79mm²). Optical assembly 250b can include spatial resolution criteria such that an acceptable depth of field includes tissue positioned within a maximum distance on either side of focal length X3, such as within 4mm on either side of focal length of focal length X3 (e.g. a depth of field of 8mm). The range-optimized embodiment of Fig. 4 can be selected if it was expected that the tissue to be measured might be positioned over a wide range of distances between the tissue and optical element 230b.

**Referring now to** **Figs. 5A and 5B****,** a perspective view and a perspective partial cross sectional view, respectively, of a sensor assembly and a rotating assembly is illustrated, consistent with the present inventive concepts. Rotating assembly 660 is operably connected to fiber assembly 200, such as has been described in detail with in reference to Fig. 1 hereabove.

Rotating assembly 660 includes motor 665 which is configured to rotate fiber assembly 200. Rotating assembly 660 can rotate fiber assembly 200 at a speed ranging from 1000rpm to 15000rpm, such as a speed between 4000 and 8000rpm, such as a speed of approximately 7260rpm. Each rotation can include a full 360° rotation or a partial rotation less than 360°, for example rotations up to 180° or up to 90°.

In some embodiments, rotating assembly 660 can be configured to rotate fiber assembly 200 with a frictionally engaged belt driven assembly as described herebelow. Various configurations can be used to rotate fiber assembly 200, such as an in-line or co-axial drive assembly; a magnetic field driven assembly; and combinations of these.

In the embodiment of Figs. 5A and 5B, rotating assembly 660 includes housing 661 which is attached to and/or maintains the relative position of one or more components of rotating assembly 660. Housing 661 can be further attached and/or maintain the position of other components of system 10, such as sensor assembly 500 and/or a translating assembly such as assembly 610 described herein. Rotating assembly 660 further includes first pulley 666, belt 667, torque assembly 670, and second pulley 671. Pulley 671 is incorporated within torque assembly 670. Torque assembly 670 further includes bearings 672, set screw 673, rotational encoder 675, a rotational encoder wheel 676, and fiber assembly coupling 680. Coupling 680 frictionally or otherwise operably engages the proximal portion of fiber assembly 200, such as to transfer rotational forces to torque shaft 205 of fiber assembly 200. Coupling 680 can be attached to fiber assembly 200 via a press-fit, adhesive or the like.

Coupling 680 is attached to housing 661 via bearings 672. Bearings 672 maintain the position of coupling 680 within housing 661, while allowing coupling 680 to freely rotate about its center axis. Bearings 672 are configured to be coaxial with coupling 680 as well as fiber assembly 200. Pulley 671 is fixedly attached to coupling 680, such as to transfer rotational forces to coupling 680. Pulley 671 can be fixedly attached to coupling 680 via one or more of set screw 673, adhesive, or the like. Rotational encoder wheel 676 is fixedly attached to coupling 680 and/or pulley 671. Rotational encoder wheel 676 maintains its angular position and velocity such that it matches the angular position and velocity of fiber assembly 200, such that the position of wheel 676 can be determined by rotational encoder 675, and that information can be transmitted to a signal processor such as signal processor 400 of Fig. 1.

Motor 665 is fixedly attached to pulley 666, such that motor 665 rotates pulley 666, rotating drive belt 667, and further rotating pulley 671 which in turn rotates torque assembly 670. Rotating assembly 660 further includes an adjustment assembly, such as at least a two dimensional adjustment mechanism, such as X-Y table 690. X-Y table 690 can be configured to fixedly attach to housing 661, such as to position housing 661 in two dimensional space. Housing 661 is fixedly attached to torque assembly 670, such X-Y table 690 can align the proximal face of optical fiber 210 with the sensor assembly 500. X-Y table 690 includes first adjustment screw 691 and second adjustment screw 692, such that first adjustment screw 691 adjusts in a first dimension and second adjustment screw 692 adjusts in a second dimension orthogonal to the first direction. Adjustment screws 691 and 692 can be used to center the proximal face of optical fiber 210, such that infrared light is collected properly by the sensor assembly 500, as described in reference to Fig. 7 herebelow.

**Referring now to** **Fig.** 6, a perspective view of a translating assembly is illustrated, consistent with the present inventive concepts. Translating assembly 610 includes motor 615, drive screw 620, translating car 625, guide 628, and linear encoder 630. Motor 615 rotates drive screw 620 such that drive screw 620 translates car 625 proximally and distally. In the embodiment of Fig. 6, drive screw 620 comprises a Yankee screw, such as is commonly used as component of a line guide in a baitcasting fishing reel. This configuration allows relatively constant speed of car 625 when motor 615 rotates at a constant velocity, and in a single rotational direction. The gears internal to car 625 allow car 625 to translate to one end of drive screw 620, where the internal gears switch position, and car 625 translates the opposite direction, to the other end of screw 620, where the gears switch back to the original orientation. In this configuration, in addition to the linear speed of car 625 being relatively uniform, reversing direction at the end of each translation is achieved relatively instantaneously. In an alternative embodiment, drive screw 620 comprises a worm drive, and the direction of travel is dependent on the rotational direction of motor 615.

Guide element 628 guides car 625 linearly such that car 625 does not rotate about drive screw 620. Guide element 628 includes linear encoder 630 which is configured to determine the linear position of car 625, and to transmit positional information to a signal processor such as signal processor 400 of Fig. 1. Car 625 includes bearings 626 which are configured to fixedly attach coupling 627 to car 625 such that coupling 627 translates with car 625. Coupling 627 is configured to be fixedly attached to fiber assembly 200, such that coupling 627 transfers linear translational forces to fiber assembly 200. Additionally, coupling 627 rotates with fiber assembly 200 as fiber assembly 200 is rotated by rotating assembly 660 as described in reference to Fig. 5A and 5B hereabove.

Translation assembly 610 further includes housings 611a, 611b, and 611c, (generally 611). Housings 611 are attached to and/or maintain the relative position of one or more components of translating assembly 610. Housings 611 can be further attached and/or maintain the position of other components of system 10, such as sensor assembly 500 or rotating assembly 660 described herein. Housing 611c is configured to fixedly attach to the proximal end of shaft 110 of probe 100, such that shaft 110 does not translate with respect to system 10, and fiber assembly 200 translates within a lumen of shaft 110.

**Referring now to** **Fig. 7****,** an optical schematic of an optical assembly proximate a sensor assembly is illustrated, consistent with the present inventive concepts. In some embodiments, optical assembly 520 can be included in a sensor assembly, such as sensor assembly 500 described herein. Optical assembly 520 can include various optical components configured to focus, split, filter, transmit without filtering (e.g. pass through), amplify, refract, reflect, polarize, or otherwise handle light such as infrared light. Typical optical components include but are not limited to: optical fiber; lens; mirror; filter; prism; amplifier; refractor; splitter; polarizer; aperture; and combinations of these. Optical assembly 520 is configured to focus IR light 40, which is received from fiber 210 as has been described herein. Optical assembly 520 includes lens 521, optical window 522, filter 523, aperture 524, and immersion lens 526. Any or all components of assembly 520 can be housed within a housing, such as sensor detector housing 501. Detector housing 501 can be a cooled housing, such as a Stirling cooled housing. Components of optical assembly 520 can include similar or dissimilar materials to the materials of optical fiber 210, such as materials configured to pass (e.g. be relatively transparent to) infrared light in the 6-15 micron wavelength range, such as light in the 8-11 micron wavelength range, as has been described herein. One or more components of assembly 520 can include anti-reflective coatings, as described in reference to Fig. 1 hereabove.

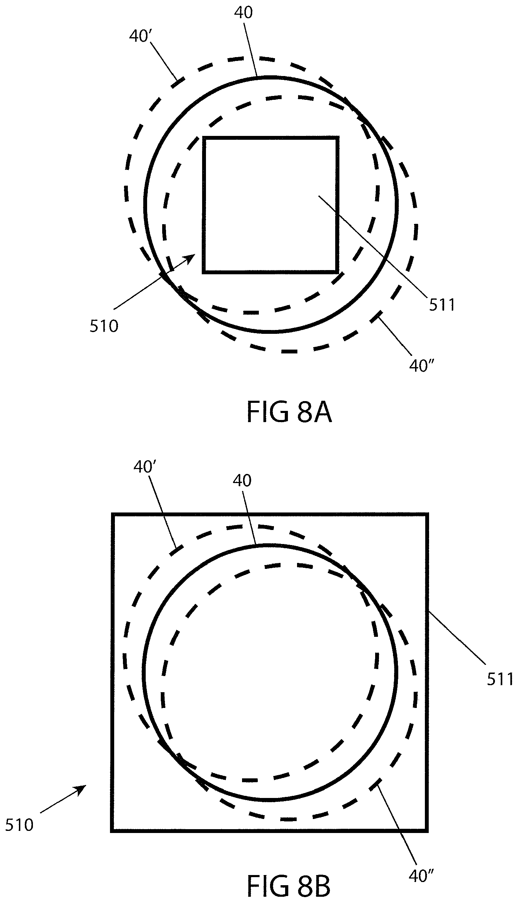

IR light 40 collected from a surface of a tissue area passes through focusing lens 521, which is configured to focus IR light 40 towards detector 510. Detector 510 includes a receiving surface, receiving surface 511, reference number not shown on Fig. 7 for clarity, but included on Figs. 8A and 8B herebelow. Fiber 210 is separated from focusing lens 521 by a physical gap, distance D1. D1 can be varied, either during use or in a manufacturing process, such as to set the magnification of IR light 40 throughout optical assembly 520. IR light 40 then passes through optical window 522, an optical component which provides a seal of a detector housing 501 (e.g. a seal that enables deep cooling of components within detector housing 501). Optical window 522 can comprise an optical component such as a planar or wedged window, a filter, or a lens configured to allow IR light 40 to pass into detector housing 501. Some or all components of assembly 520 can be enclosed within detector housing 501, including IR detector 510, such as when detector housing 501 comprises a cooled housing and the enclosed components are cooled to the temperature in detector housing 501. Cooling of the components within detector housing 501 minimizes the amount of infrared light emitted by the components, and thus increasing the signal to noise ratio of the system. In some embodiments, components within detector housing 501 are cooled to approximately 77 degrees Kelvin. Filter 523 comprises an optical filter configured to pass IR light 40, for example infrared light comprising a wavelength of between 8 and 11 microns. All other wavelengths are blocked, or partially blocked by filter 523, increasing the signal to noise ratio of the system.

Assembly 520 further includes a cold aperture, aperture 524, configured to block infrared light from outside the desired field of view from reaching detector 510. Immersion lens 526 further focuses IR light 40 onto the face of detector 510. Immersion lens 526 allows optical assembly 520 to incorporate a smaller light-receiving surface of detector 510 without increasing the overall length of the system. Reducing the light receiving surface area of detector 510 can provide improved signal to noise and/or temporal performance (e.g. faster response time). Furthermore, the shape of detector 510 can optimally be matched to the core shape of fiber 210 (e.g. example round or square) such as to minimize the area of detector 510 that is unlikely to receive IR Light 40. IR light 40 emitted onto the face of detector 510 can be converted into a voltage signal, as described in reference to Fig. 1 hereabove, and converted to a table of temperature values versus tissue area locations, which can be displayed as a temperature map as described herein. In some embodiments, the voltage signal represents a change in IR light 40 received by detector 510 (i.e. a differential signal).

The optical pathway of Fig. 7 can be constructed and arranged to relatively fill, "overfill" or "underfill" the receiving surface of detector 510 with IR light 40, such as is described herebelow in reference to Figs. 8A and 8B, respectively.

Fig. 8A is an optical schematic of an infrared detector illustrating the projections of infrared light focused toward the detector, in a configuration that overfills the detector, consistent with the present inventive concepts. Detector 510 can be similar to detector 510 of Fig. 7 described hereabove, and includes a receiving surface 511 constructed and arranged to receive infrared light such that detector 510 can convert the received infrared light into a signal. IR light 40 can represent the projection of infrared light focused towards detector 510, such as by an optical assembly, such as optical assembly 520 of Fig. 7. In the illustrated embodiment, detector 510 is "overfilled", such that projections of received infrared light (e.g. light received from fiber 210 of Fig. 7) fully cover, and potentially extend beyond, the receiving surface 511 of detector 510. IR light 40, 40' and 40" represent projections that have a cross sectional area greater than surface 511 (e.g. due to the magnification that results from optical assembly 520 of Fig. 7). IR light 40 is relatively centered about surface 511. IR light 40' and 40" can represent a precession of light 40 away from its centered position, such as a precession caused by one or more of: static alignment or misalignment of one or more optical components; irregular rotation of an optical fiber or other rotating component of the system; or another cause.

**Fig. 8B** is an optical schematic of an infrared detector illustrating the projections of infrared light focused toward the detector, in a configuration that underfills the detector, consistent with the present inventive concepts. Detector 510 can be similar to detector 510 of Fig. 7 described hereabove. IR light 40 can represent the projection of infrared light focused towards detector 510 (e.g. from a fiber such as fiber 210 of Fig. 7), such as by an optical assembly, such as optical assembly 520 of Fig. 7. In the illustrated embodiment, detector 510 is "underfilled", such that projections of received infrared light partially cover the receiving surface 511 of detector 510. IR light 40, 40' and 40" represent projections have a cross sectional area less than surface 511 (e.g. due to the magnification that results optical assembly 520 of Fig. 7). IR light 40 is relatively centered about surface 511. IR light 40' and 40" can represent a precession of IR light 40 away from its centered position, such as a precession caused by one or more of: static alignment or misalignment of one or more optical components; irregular rotation of an optical fiber or other rotating component of the system; or another cause. In some embodiments, a proximal optical assembly (e.g. optical assembly 520 of Fig. 7) can be constructed and arranged such that all anticipated precessions of IR light 40 (e.g. IR light 40' and 40") are fully received by (e.g. do not extent beyond) surface 511.

In some embodiments, the overfill design of Fig. 8A is selected, such as to minimize infrared light emanating from objects or surfaces other than the proximal end of fiber 210 from being received by surface 511; minimize errors that result from misalignment, non-uniform rotation or other abnormalities that can cause light emanating from the proximal end of fiber 210 to move onto and/or off of surface 511; and combinations thereof. In other embodiments, the underfill design of Fig. 8B is selected, such as to maximize the amount of light received by surface 511 that emanates from the proximal end of fiber 210. In some embodiments, optical assembly 520 is constructed and arranged to relatively, completely "fill" detector 510, such that the size of the projected light onto surface 511 relatively matches the size of surface 511. In some embodiment, the system of the present inventive concepts is constructed and arranged to allow an operator to change the amount of fill or overfill of infrared light received on surface 511, such as by adjusting the magnification of optical assembly 520 as has been described hereabove.

In the embodiments of Figs. 8A and 8B, receiving surface 511 of detector 510 comprises a square infrared light receiving surface. In other embodiments, surface 511 can comprise a surface with a shape selected from the group consisting of: circular; elliptical; rectangular; trapezoidal; triangular; and combinations of these. In some embodiments, receiving surface 511 comprises a shape configured to match an optical component of the system, such as the cross sectional shape of an optical fiber (e.g. optical fiber 210 of Fig. 7), or the shape of the projection of infrared light from a lens (e.g. focusing lens 521 of Fig. 7). In some embodiments, optical assembly 520 is constructed and arranged to project IR light 40 onto surface 511 in a circular, elliptical, rectangular or square pattern, such as when surface 511 comprises a circular, elliptical, rectangular or square pattern, respectively.

## Claims

1. A system for producing surface temperature estimations of a tissue surface, comprising:
a probe comprising a first optical assembly constructed and arranged to receive infrared light emitted from multiple tissue surface areas and a fiber comprising a proximal end and a distal end, wherein the distal end is optically coupled to receive infrared light from the first optical assembly;
an infrared sensor optically coupled to the fiber proximal end, which is constructed and arranged to produce a signal that correlates to an average temperature of each of the multiple tissue surface areas, and a shaft, wherein the fiber is slidingly received by the shaft
**characterized in that** the shaft includes the at least one band, wherein the first optical assembly collects infrared light emanating from the at least one band and at least one temperature sensor is constructed and arranged to measure a temperature of the at least one band to calibrate the infrared sensor.

2. The system of claim 1, wherein the system is constructed and arranged to produce surface temperature estimations of a surface of an esophagus.

3. The system of claim 1 or 2, wherein the probe has a diameter less than or equal to 15Fr.

4. The system according to at least one of the preceding claims, wherein the first optical assembly comprises a surrounding tube, and the first optical assembly comprises an optical element selected from the group consisting of: optical fiber; lens; mirror; filter; prism; amplifier; retractor; splitter; polarizer; or an aperture, and the first optical assembly comprises an optical element constructed and arranged to perform an action on the received infrared light and the first optical assembly comprises a rigid length less than or equal to 3cm and the first optical assembly comprises an optical element comprising a planar surface, an angled surface and a convex surface and the first optical assembly comprises an optical element with a first surface constructed and arranged to receive infrared light from tissue and a second surface constructed and arranged to direct the received infrared light to the fiber distal end and the first optical assembly comprises an optical separation distance between the second surface and the fiber distal end and the system is constructed and the system comprises spatial resolution criteria correlating to a depth of field of between 1.5mm and 10mm.

5. The system according to at least one of the preceding claims, wherein the multiple tissue surface areas comprise multiple tissue surfaces, each comprising an area between 0.1 mm² and 20mm².

6. The system according to at least one of the preceding claims, wherein the fiber comprises a material selected from the group consisting of: zinc selenide, germanium; germanium oxide, silver halide; chalcogenide; a hollow core fiber material; and combinations thereof, and the fiber comprises a material relatively transmissive to wavelengths between 6µm and 15µm and the fiber comprises at least one anti-reflective coating and the fiber further comprises a twist resisting structure surrounding at least a portion of the fiber between the fiber proximal end and the fiber distal end and the twist resisting structure comprises a torque shaft.

7. The system according to at least one of the preceding claims, wherein the system is constructed and arranged to perform a manipulation on the fiber selected from the group consisting of: rotating the fiber; translating the fiber; and combinations thereof.

8. The system according to at least one of the preceding claims, wherein the sensor comprises an infrared light detector and the sensor comprises a response time less than or equal to 200 milliseconds and the sensor comprises a cooling assembly constructed and arranged to cool one or more portions of the sensor and the signal represents a change in received infrared light.

9. The system according to at least one of the preceding claims in particular wherein the shaft comprises a rounded tip and the shaft comprises a material selected from the group consisting of: polyethylene; polyimide; polyurethane; polyether block amide; and combinations thereof and the shaft comprises a braided shaft and the shaft is constructed and arranged for over-the-wire insertion into a body lumen and the shaft is constructed and arranged for insertion into a nostril and the shaft is constructed and arranged to be inserted through anatomy with a radius of curvature less than or equal to 4 inches.

10. The system according to at least one of the preceding claims, further comprising a second optical assembly constructed and arranged to receive infrared light from the fiber and direct light onto a receiving surface of the sensor.

11. The system according to at least one of the preceding claims further comprising a rotating assembly and wherein the rotating assembly is constructed and arranged to rotate the first optical assembly and further comprising a translating assembly constructed and arranged to translate the fiber.

12. The system according to at least one of the preceding claims further comprising a translating assembly constructed and arranged to translate the fiber and the translating assembly is constructed and arranged to translate the fiber in a reciprocating motion and further comprising a rotating assembly constructed and arranged to rotate the fiber and the system is constructed and arranged to simultaneously rotate and translate the fiber or the system is constructed and arranged to sequentially rotate and translate the fiber.

13. The system according to at least one of the preceding claims, further comprising a user interface constructed and arranged to display a graphical temperature map of the average temperature of each of the multiple tissue surface areas.

14. The system according to at least one of the preceding claims, further comprising a signal processing unit, in particular wherein the signal processing unit is constructed and arranged to correlate the sensor signal into a table of temperature values correlating to the multiple tissue surface areas.

15. The system according to at least one of the preceding claims, wherein the at least one band comprises a proximal band, wherein the first optical assembly is constructed and arranged to translate between a proximal position and distal position, and wherein the proximal band is positioned relative the proximal position and the at least one band comprises a distal band, wherein the first optical assembly is constructed and arranged to translate between a proximal position and distal position.

16. The system according to at least one of the preceding claims, further comprising a positioning member constructed and arranged to position the first optical assembly at a distance from the tissue surface and the positioning member is constructed and arranged to center the first optical assembly in a body lumen.

## Patentansprüche

1. System zum Erzeugen von Oberflächentemperaturschätzungen bei einer Gewebeoberfläche, welches aufweist:
eine Sonde, aufweisend eine erste optische Anordnung, die gebildet und angeordnet ist zum Empfangen von von mehreren Gewebeoberflächenbereichen emittiertem Infrarotlicht und eine Faser aufweisend ein proximales Ende und ein distales Ende, wobei das distale Ende optisch gekoppelt ist, um Infrarotlicht von der ersten optischen Anordnung zu empfangen;
einen Infrarotsensor, der optisch mit dem proximalen Ende der Faser gekoppelt ist, der gebildet und angeordnet ist, ein Signal zu erzeugen, das mit einer durchschnittlichen Temperatur von jedem der mehreren Gewebeoberflächenbereiche korreliert, und
eine Welle, wobei die Faser gleitend von der Welle aufgenommen ist, **dadurch gekennzeichnet, dass**
die Welle das zumindest eine Band enthält, wobei die erste optische Anordnung aus dem zumindest einen Band austretendes Infrarotlicht sammelt, und zumindest ein Temperatursensor so gebildet und angeordnet ist, eine Temperatur des zumindest einen Bandes zu messen, um den Infrarotsensor zu kalibrieren.

2. System nach Anspruch 1, bei dem das System gestaltet und angeordnet ist, Oberflächentemperaturschätzungen bei einer Oberfläche einer Speiseröhre zu erzeugen.

3. System nach Anspruch 1 oder 2, bei dem die Sonde einen Durchmesser hat, der kleiner als oder gleich 15 Fr ist.

4. System nach zumindest einem der vorhergehenden Ansprüche, bei dem die erste optische Anordnung ein umgebendes Rohr aufweist und die erste optische Anordnung ein optisches Element aufweist, das ausgewählt ist aus der Gruppe bestehend aus: optische Faser; Linse; Spiegel; Filter; Prisma; Verstärker; Retraktor; Teiler; Polarisator; oder eine Öffnung, und die erste optische Anordnung ein optisches Element aufweist, das gestaltet und angeordnet ist zum Durchführen einer Aktion bei dem empfangenen Infrarotlicht, und die erste optische Anordnung eine starre Länge aufweist, die kürzer als oder gleich 3 cm ist, und die erste optische Anordnung ein optisches Element aufweist, das eine ebene Oberfläche, eine abgewinkelte Oberfläche und eine konvexe Oberfläche aufweist, und die erste optische Anordnung ein optisches Element mit einer ersten Oberfläche, die gestaltet und angeordnet ist, Infrarotlicht von Gewebe zu empfangen, und eine zweite Oberfläche, die gestaltet und angeordnet ist, das empfangene Infrarotlicht zu dem distalen Faserende zu leiten, aufweist, und die erste optische Anordnung einen optischen Trennabstand zwischen der zweiten Oberfläche und dem distalen Faserende aufweist und das System gestaltet ist und das System räumliche Auflösungskriterien aufweist, die zu einer Feldtiefe zwischen 1,5 mm und 10 mm korrelieren.

5. System nach zumindest einem der vorhergehenden Ansprüche, bei dem die mehreren Gewebeoberflächenbereiche mehrere Gewebeoberflächen aufweisen, die jeweils eine Fläche zwischen 0,1 mm² und 20 mm² aufweisen.

6. System nach zumindest einem der vorhergehenden Ansprüche, bei dem die Faser ein Material aufweist, das ausgewählt ist aus der Gruppe bestehend aus: Zinkselenid, Germanium; Germaniumoxid, Silberhalogenid; Chalcogen; ein Fasermaterial mit hohlem Kern; und Kombinationen hiervon, und die Faser ein Material aufweist, das relativ durchlässig für Wellenlängen zwischen 6 µm und 15 µm ist, und die Faser zumindest eine Antireflexionsbeschichtung aufweist, und die Faser weiterhin eine einer Verdrehung widerstehende Struktur aufweist, die zumindest einen Bereich der Faser zwischen dem proximalen Faserende und dem distalen Faserende umgibt, und die einer Verdrehung widerstehende Struktur eine Drehmomentwelle aufweist.

7. System nach zumindest einem der vorhergehenden Ansprüche, bei dem das System gestaltet und angeordnet ist, eine Behandlung der Faser durchzuführen, die ausgewählt ist aus der Gruppe bestehend aus: Drehen der Faser; Verschieben der Faser; und Kombinationen hiervon.

8. System nach zumindest einem der vorhergehenden Ansprüche, bei dem der Sensor einen Infrarotlichtdetektor aufweist und der Sensor eine Ansprechzeit von weniger als oder gleich 200 Millisekunden aufweist, und der Sensor eine Kühlanordnung aufweist, die gestaltet und angeordnet ist, einen oder mehrere Bereiche des Sensors zu kühlen, und das Signal eine Änderung des empfangenen Infrarotlichts darstellt.

9. System nach zumindest einem der vorhergehenden Ansprüche, insbesondere bei denen die Welle eine gerundete Spitze aufweist und die Welle ein Material aufweist, das ausgewählt ist aus der Gruppe bestehend aus: Polyethylen; Polyimid; Polyurethan; Polyetherblockamid; und Kombinationen hiervon, und die Welle eine geflochtene Welle aufweist und die Welle gestaltet und angeordnet ist für eine Über-den-Draht-Einführung in ein Körperlumen, und die Welle gestaltet und angeordnet ist für die Einführung in ein Nasenloch, und die Welle gestaltet und angeordnet ist, durch eine Anatomie mit einem Krümmungsradius von weniger als oder gleich 4 Zoll eingeführt zu werden.

10. System nach zumindest einem der vorhergehenden Ansprüche, weiterhin aufweisend eine zweite optische Anordnung, die gestaltet und angeordnet ist, Infrarotlicht von der Faser und direktes Licht auf einer Empfangsfläche des Sensors zu empfangen.

11. System nach zumindest einem der vorhergehenden Ansprüche, weiterhin aufweisend eine Drehanordnung, wobei die Drehanordnung gestaltet und angeordnet ist, die erste optische Anordnung zu drehen, und weiterhin eine Verschiebungsanordnung aufweist, die zum Verschieben der Faser gestaltet und angeordnet ist.

12. System nach zumindest einem der vorhergehenden Ansprüche, weiterhin aufweisend eine Verschiebeanordnung, die gestaltet und angeordnet ist, die Faser zu verschieben, und die Verschiebeanordnung gestaltet und angeordnet ist, die Faser in einer hin- und hergehenden Bewegung zu verschieben, und weiterhin aufweisend eine Drehanordnung, die gestaltet und angeordnet ist, die Faser zu drehen, und das System gestaltet und angeordnet ist, die Faser gleichzeitig zu drehen und zu verschieben, oder das System gestaltet und angeordnet ist, die Faser nacheinander zu drehen und zu verschieben.

13. System nach zumindest einem der vorhergehenden Ansprüche, weiterhin aufweisend eine Benutzerschnittstelle, die gestaltet und angeordnet ist, eine grafische Temperaturkarte der Durchschnittstemperatur jedes der mehreren Gewebeoberflächenbereiche anzuzeigen.

14. System nach zumindest einem der vorhergehenden Ansprüche, weiterhin aufweisend eine Signalverarbeitungseinheit, insbesondere bei dem die Signalverarbeitungseinheit gestaltet und angeordnet ist, das Sensorsignal in eine Tabelle von Temperaturwerten, die mit den mehreren Gewebeoberflächenbereichen korreliert sind, zu korrelieren.

15. System nach zumindest einem der vorhergehenden Ansprüche, bei dem das zumindest eine Band ein proximales Band aufweist, wobei die erste optische Anordnung gestaltet und angeordnet ist, zwischen einer proximalen Position und einer distalen Position verschoben zu werden, und wobei das proximale Band relativ zu der proximalen Position positioniert ist, und das zumindest eine Band ein distales Band aufweist, wobei die erste optische Anordnung gestaltet und angeordnet ist, zwischen der proximalen Position und der distalen Position verschoben zu werden.

16. System nach zumindest einem der vorhergehenden Ansprüche, weiterhin aufweisend ein Positionierungsteil, das gestaltet und angeordnet ist, die erste optische Anordnung in einem Abstand von der Gewebeoberfläche zu positionieren, und das Positionierungsteil gestaltet und angeordnet ist, die erste optische Anordnung in einem Körperlumen zu zentrieren.

## Revendications

1. Système pour produire des estimations de température de surface d'une surface de tissu, comprenant :
une sonde qui comprend un premier assemblage optique qui est construit et agencé de manière à ce qu'il reçoive une lumière infrarouge qui est émise depuis de multiples zones de surface de tissu et une fibre qui comprend une extrémité proximale et une extrémité distale, dans lequel l'extrémité distale est couplée optiquement de manière à ce qu'elle reçoive une lumière infrarouge qui provient du premier assemblage optique ;
un capteur infrarouge qui est couplé optiquement à l'extrémité proximale de la fibre, lequel capteur infrarouge est construit et agencé de manière à ce qu'il produise un signal qui est corrélé à une température moyenne de chacune des multiples zones de surface de tissu ; et
un arbre, dans lequel la fibre est reçue de façon coulissante par l'arbre ;
**caractérisé en ce que** :
l'arbre inclut l'au moins une bande, dans lequel le premier assemblage optique collecte la lumière infrarouge qui émane de l'au moins une bande, et au moins un capteur de température est construit et agencé de manière à ce qu'il mesure une température de l'au moins une bande de manière à étalonner le capteur infrarouge.

2. Système selon la revendication 1, dans lequel le système est construit et agencé de manière à ce qu'il produise des estimations de température de surface d'une surface d'un oesophage.

3. Système selon la revendication 1 ou 2, dans lequel la sonde présente un diamètre qui est inférieur ou égal à 15 Fr.

4. Système selon au moins l'une des revendications qui précèdent, dans lequel le premier assemblage optique comprend un tube d'entourage enveloppant, et le premier assemblage optique comprend un élément optique qui est sélectionné parmi le groupe qui est constitué par : une fibre optique ; un objectif ; un miroir ; un filtre ; un prisme ; un amplificateur ; un rétracteur ; un séparateur ; un polariseur ; ou une ouverture, et le premier assemblage optique comprend un élément optique qui est construit et agencé de manière à ce qu'il réalise une action sur la lumière infrarouge reçue, et le premier assemblage optique comprend une longueur rigide inférieure ou égale à 3 cm et le premier assemblage optique comprend un élément optique qui comprend une surface plane, une surface angulée et une surface convexe et le premier assemblage optique comprend un élément optique qui est muni d'une première surface qui est construite et agencée de manière à ce qu'elle reçoive une lumière infrarouge en provenance du tissu et d'une seconde surface qui est construite et agencée de manière à ce qu'elle dirige la lumière infrarouge reçue sur l'extrémité distale de la fibre et le premier assemblage optique comprend une distance de séparation optique entre la seconde surface et l'extrémité distale de la fibre et le système comprend un critère de résolution spatiale qui est corrélé à une profondeur de champ entre 1,5 mm et 10 mm.

5. Système selon au moins l'une des revendications qui précèdent, dans lequel les multiples zones de surface de tissu comprennent de multiples surfaces de tissu, chacune présentant une aire entre 0,1 mm² et 20 mm².

6. Système selon au moins l'une des revendications qui précèdent, dans lequel la fibre comprend un matériau qui est sélectionné parmi le groupe qui est constitué par : le séléniure de zinc ; le germanium ; l'oxyde de germanium ; l'halogénure d'argent ; un chalcogénure ; un matériau de fibre à âme creuse ; et des combinaisons afférentes, et la fibre comprend un matériau relativement transmissif vis-à-vis de longueurs d'onde entre 6 µm et 15 µm et la fibre comprend au moins un revêtement antireflet et la fibre comprend en outre une structure résistant à la torsion qui entoure au moins une partie de la fibre entre l'extrémité proximale de la fibre et l'extrémité distale de la fibre et la structure résistant à la torsion comprend un arbre de couple.

7. Système selon au moins l'une des revendications qui précèdent, dans lequel le système est construit et agencé de manière à ce qu'il réalise une manipulation sur la fibre qui est sélectionnée parmi le groupe qui est constitué par : une rotation de la fibre ; une translation de la fibre ; et des combinaisons afférentes.

8. Système selon au moins l'une des revendications qui précèdent, dans lequel le capteur comprend un détecteur de lumière infrarouge et le capteur présente un temps de réponse inférieur ou égal à 200 millisecondes et le capteur comprend un assemblage de refroidissement qui est construit et agencé de manière à ce qu'il refroidisse une ou plusieurs partie(s) du capteur et le signal représente une variation de la lumière infrarouge reçue.

9. Système selon au moins l'une des revendications qui précèdent, en particulier dans lequel l'arbre comprend une pointe d'extrémité arrondie et l'arbre comprend un matériau qui est sélectionné parmi le groupe qui est constitué par : le polyéthylène ; le polyimide ; le polyuréthane ; le polyéther bloc amide ; et des combinaisons afférentes, et l'arbre comprend un arbre tressé et l'arbre est construit et agencé dans le but de son insertion au-dessus du fil à l'intérieur d'une lumière du corps et l'arbre est construit et agencé dans le but de son insertion à l'intérieur d'une narine et l'arbre est construit et agencé de manière à ce qu'il soit inséré au travers d'une anatomie qui présente un rayon de courbure inférieur ou égal à 4 pouces.

10. Système selon au moins l'une des revendications qui précèdent, comprenant en outre un second assemblage optique qui est construit et agencé de manière à ce qu'il reçoive une lumière infrarouge en provenance de la fibre et de manière à ce qu'il dirige la lumière infrarouge sur une surface de réception du capteur.

11. Système selon au moins l'une des revendications qui précèdent, comprenant en outre un assemblage de rotation et dans lequel l'assemblage de rotation est construit et agencé de manière à ce qu'il entraîne en rotation le premier assemblage optique et comprenant en outre un assemblage de translation qui est construit et agencé de manière à ce qu'il translate la fibre.

12. Système selon au moins l'une des revendications qui précèdent, comprenant en outre un assemblage de translation qui est construit et agencé de manière à ce qu'il translate la fibre et l'assemblage de translation est construit et agencé de manière à ce qu'il translate la fibre selon un déplacement en va-et-vient et comprenant en outre un assemblage de rotation qui est construit et agencé de manière à ce qu'il entraîne en rotation la fibre et le système est construit et agencé de manière à ce que, de façon simultanée, il entraîne en rotation et translate la fibre ou le système est construit et agencé de manière à ce que, de façon séquentielle, il entraîne en rotation et translate la fibre.

13. Système selon au moins l'une des revendications qui précèdent, comprenant en outre une interface utilisateur qui est construite et agencée de manière à ce qu'elle affiche une carte de températures graphique de la température moyenne de chacune des multiples zones de surface de tissu.

14. Système selon au moins l'une des revendications qui précèdent, comprenant en outre une unité de traitement de signal, en particulier dans lequel l'unité de traitement de signal est construite et agencée de manière à ce qu'elle réalise une corrélation du signal de capteur à l'intérieur d'une table de valeurs de température qui sont corrélées aux multiples zones de surface de tissu.

15. Système selon au moins l'une des revendications qui précèdent, dans lequel l'au moins une bande comprend une bande proximale, dans lequel le premier assemblage optique est construit et agencé de manière à ce qu'il réalise une translation entre une position proximale et une position distale, et dans lequel la bande proximale est positionnée par rapport à la position proximale et l'au moins une bande comprend une bande distale, dans lequel le premier assemblage optique est construit et agencé de manière à ce qu'il réalise une translation entre une position proximale et une position distale.

16. Système selon au moins l'une des revendications qui précèdent, comprenant en outre un élément de positionnement qui est construit et agencé de manière à ce qu'il positionne le premier assemblage optique à une distance de la surface de tissu et l'élément de positionnement est construit et agencé de manière à ce qu'il centre le premier assemblage optique dans une lumière du corps.
